(19) Europäisches Patentamt
European Patent Office
Office européen des brevets

(11) EP 0 793 481 B1

(12) EUROPEAN PATENT SPECIFICATION

(45) Date of publication and mention of the grant of the patent:
**02.06.2004 Bulletin 2004/23**

(21) Application number: **94926177.0**

(22) Date of filing: **10.08.1994**

(51) Int Cl.[7]: **A61K 7/48**

(86) International application number:
**PCT/EP1994/002665**

(87) International publication number:
**WO 1995/005153 (23.02.1995 Gazette 1995/09)**

(54) **COSMETIC COMPOSITION CONTAINING HYDROXY ALKANOATE DERIVATIVES**

KOSMETISCHE ZUSAMMENSETZUNG ENTHALTEND HYDROXYALKANOAT DERIVATE

COMPOSITION COSMETIQUE CONTENANT DES DERIVES D'HYDROXY-ALCANOATE

(84) Designated Contracting States:
**CH DE ES FR GB IT LI NL SE**

(30) Priority: **13.08.1993 GB 9316848**
**25.08.1993 GB 9317684**

(43) Date of publication of application:
**10.09.1997 Bulletin 1997/37**

(73) Proprietors:
- **UNILEVER PLC**
**London EC4P 4BQ (GB)**
Designated Contracting States:
**GB**
- **UNILEVER N.V.**
**3013 AL Rotterdam (NL)**
Designated Contracting States:
**CH DE ES FR IT LI NL SE**

(72) Inventors:
- **BROWSER, Paul Anthony**
**Gayton, Wirral, Merseyside L60 3RW (GB)**
- **EDWARDS, Christopher John C.**
**Wetherby, West Yorkshire LS22 5BU (GB)**
- **GRIEVESON, Ailsa Pauline H.**
**Wirral, Merseyside L63 3DJ (GB)**
- **LYLE, Ian Gardner**
**Deeside, Clwyd CH5 1XQ (GB)**
- **ROSSER, David Arthur**
**Wirral, Merseyside L60 4RD (GB)**
- **SCOTT, Ian Richard**
**Allendale, NJ 07401 (US)**

(74) Representative: **Elliott, Peter William et al**
**Unilever plc**
**Patent Division**
**Colworth House**
**Sharnbrook**
**Bedford MK44 1LQ (GB)**

(56) References cited:

| | |
|---|---|
| **EP-A- 0 007 785** | **EP-A- 0 150 914** |
| **EP-A- 0 595 528** | **WO-A-88/06880** |
| **WO-A-91/01713** | **DE-A- 4 215 502** |
| **DE-C- 4 215 501** | **DE-U- 9 206 337** |
| **US-A- 4 105 783** | **US-A- 4 198 311** |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 793 481 B1

Printed by Jouve, 75001 PARIS (FR)

## Description

### FIELD OF THE INVENTION

The invention relates to the topical use of hydroxy alkanoate derivatives for improving the condition of human skin, particularly for moisturising the skin, and for reducing skin drying and for delaying the appearance of or eliminating wrinkles and fine lines on the skin.

The invention also relates to a method for the delivery of a hydroxyalkanoate for moisturising the skin and to compositions for topical use to achieve this benefit.

### BACKGROUND TO THE INVENTION

Human skin consists essentially of two layers: the inner dermis and the outer epidermis, the former functioning mainly as a mechanical support for the latter.

The epidermis, which can be as little as 0.06 mm thick in the case of the eye lid to as much as 0.8 mm on the foot, itself comprises four or five layers, namely:

(i) the Stratum Malpigii, which is the germinative layer of cells at the base of the epidermis that adjoins the Dermis.

(ii) the Stratum Spinosum, the prickle cell layer which represents the first morphologically distinct stage in the differentiation of epidermal cells. It consists of numerous evenly spaced intercellular bridges - tonofilaments - each with a central thickening. The margins of several of these thickenings accounts for the appearance of desmosomes. The tonofilaments form the earliest precursor of keratin.

(iii) the Stratum Granulosum, the granular layer immediately above the prickle cell layer, which contains basophilic granules of keratohyalin. Also present in the Stratum Granulosum are the bridges (desmosomes and tono filaments seen in the prickle cell layers), but their close apposition renders them less visible.

(iv) the Stratum Lucidum, seen especially in the epidermis of the hand and foot, comprises cells which are of even thickness and essentially non-nuclear.

(v) the Stratum Corneum, which lies above the Stratum Lucidum (when present), forms the outermost layer of the epidermis. The Stratum Corneum is composed of dead, flat, fully keratinized cells which lie on top of one another to a depth of from 0.02 to 0.8 mm. The Stratum corneum also possesses lipid materials which effectively form a waterproof barrier to the external surface of the skin.

Beneath the epidermis is the dermis which is composed of collagen, usually accompanied by elastin and reticulin. These materials are fibrous proteins embedded in a mucopolysaccharide ground substance. Several cellular types, together with nervous and vascular networks, are found in the dermis, together with specialised appendages, including sweat glands, hair follicles with associated sebaceous glands.

A soft, supple and flexible skin has a marked cosmetic appeal and these characteristics are attributes of normal functioning epidermis, particularly with respect to the young human subject. The outer layer of the epidermis, i.e. the stratum corneum, can however become dry and flaky following exposure to adverse climatic conditions, or by excessive contact with detergents or solvents which results in a loss of skin moisture. Consequently, the skin can lose its soft, supple and flexible characteristics.

Emollients such as fats, phospholipids and stearols, have in the past been used to soften dry skin, but this can leave the skin greasy and unattractive. As an alternative, the topical application to the skin of classical humectants does not alleviate this problem, as these compounds are not particularly skin substantive and are generally rinsed from the skin during washing.

It is therefore apparent that there exists a continuing need for effective methods for treating dry flaky skin to restore its original soft, supple and flexible characteristics, and indeed for maintaining these attributes of normal functioning epidermis.

In an article by Baiocchi et al in Cosmetics and Perfumery 90, 31-34 (1975), it is stated that sodium stearoyl lactylate, when incorporated in a hand cream or lotion, results in a subjectively smooth and supple but not excessively greasy feeling when such creams or lotions are topically applied to the hands. However, the primary reason for including this lactylate in such formulations is to function as a very efficient emulsifier.

In an article by Osipow et al in Drug & Cosmet Ind, May 1969, 64ff, it is disclosed that sodium stearoyl lactylate may be used in oil-in-water cosmetic creams as the emulsifier to impart body, lubricity and opalescence to the cream.

It is alleged that its absorption to the skin may enhance its softening action.

In another article by Murphy in Cosmetics and Toiletries 94, 43ff (1979), the sorption of acyl lactylates on the skin was examined by using pigskin as a model. It is described that sodium isostearoyl lactylate appears to reduce dryness and scaling of skin and restores a healthy texture to dry skin.

Murphy in Cosmetics and Toiletries 93, 31 (1978) discusses a systemable approach to skin moisturisation and concludes that a combination of the pyrrolidone carboxylic acid (PCA) sodium salt, sodium lactate and lactic acid can be used as effective humectants to hold moisture in the skin.

US 4,105, 783 (Yu & Van Scott) discloses a therapeutic treatment of dry skin consisting of the topical application of a lotion, cream or ointment containing one or more $\alpha$ or $\beta$- hydroxy acids including glycolic acid and lactic acid.

EP 0 530 866 (Unilever) concerns novel sulfoxy alkanoates surfactants which it is believed are broken down by enzymes naturally present in the skin, or are naturally hydrolysed upon contact with the skin to yield "Benefit Reagents" e.g. Hydroxy acid and/or Fatty Alcohol.

EP 0 442 708 (Unilever) discloses cosmetic compositions containing 2-hydroxy alkanoic acids. Due to the presence of these acids in compositions, several benefits are imparted to the skin, such as an increase in the elasticity of the skin, particularly of the stratum corneum. Similarly, EP 0 007 785 (Unilever) discloses cosmetic compositions comprising 2-hydroxy alkanoic acids, which also give various skin benefits when topically applied to the skin.

WO 91/01713 (R.I.T.A. Corporation) discloses a cosmetic base composition which exhibits therapeutic properties comprising an acyl fatty acid alpha-hydroxy carboxylic acid ester or alkali metal salt, a sucrose fatty acid ester and a solvent. WO 88/0688 assigned to the same corporation, also teaches compositions of this type.

US 4,198,311 (C.J. Patterson Company) concerns a toilet bar composed of a cleansing agent and an alkali metal salt of an acyl lactylate or glycolate. The lactylate or glycolate conditions the skin and imparts a pleasing feel to the skin.

EP 0 595 528 (Unilever) relevant to the patent as prior art under Article 54(3) EPC, teaches the use of acyl lactylates as skin elasticity enhancing agents, in particular these compounds have been found to increase the elasticity of the stratum corneum.

However, the extent to which the moisturisation of skin, or its ability to remain moist without becoming dry, by topical application of so-called "moisturisers" as proposed by other workers in the field of cosmetic science, is not significant. A search has therefore been conducted for other active materials that can be employed in this way for enhancing the moisturisation of skin or, once moisturised, for restricting the extent to which skin moisture loss will occur.

It has long been recognised that cream or lotion formulations containing lactic acid, usually as lactate ions in products near neutral pH, when applied topically to the skin, can improve the flexibility and texture of the skin, and it is believed that lactate contributed to this effect. In studying this approach, we have applied such creams and lotions to the skin and have shown that although lactate can thereby be deposited on the surface of the stratum corneum which forms the outermost part of the skin, very little actually penetrates through the stratum corneum to the underlying regions of the epidermis, namely to the Stratum Granulosum and other strata below. This is thought to be due to the hydrophilic (i.e. lipophobic) property of lactate ions which renders them relatively incompatible with the lipids naturally present in the Stratum Corneum, and which thereby present a barrier to the adsorption of hydrophilic molecules.

While investigating the properties of derivatives of lactic acid that were more lipophilic than lactate itself, (e.g. sodium lactate) we discovered that a range of acyl lactylates were more readily adsorbed on contact with the skin and indeed migrated through the skin to reach the epidermis beneath the Stratum Corneum at a rate and to an extent that was far superior to lactate ions. We also made the discovery using labelled materials and radio tracer techniques, that these acyl lactylate molecules were cleaved within the epidermis, most likely by the presence of endogenous esterases or other enzymes, to form lactate ions deep in the epidermis, as far as the Stratum Malpigii.

We extended our study of acyl lactylates to acyl glycolates, and we were again able to show that molecular cleavage occurred in the epidermis, following topical application of these molecules, to release in situ the corresponding hydroxy acid. This shorter chain length hydroxy acid was also shown to possess properties which enhanced the moisturisation of skin and/or reduced the extent to which moisture is lost from the skin.

The invention is accordingly concerned with the generation in situ in the epidermis of lactic or glycolic acids and the topical use of their corresponding acyl or alkyl derivatives to achieve this end.

## DEFINITION OF THE INVENTION

Accordingly, the invention provides for the use of an effective amount of at least 0.001% by weight based on the total composition of a hydroxy alkanoate derivative having the structure (1):

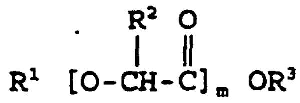 (1)

where

$R^1$ represents H- or

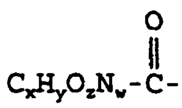

$R^2$ represents H- or $-CH_3$,
$R^3$ represents H-, $C_xH_yO_zN_w$- or a metallic, ammonium or alkanolammonium counterion,
x is an integer of from 1 to 20,
y is an integer of from 3 to 41,
z is 0, or an integer of from 1 to 10,
w is 0, or an integer of from 1 to 5, and
m is an integer of from 1 to 5

provided that when $R^1$ is H-, then $R^3$ is $C_xH_yO_zN_w$-, or when

$R^3$ is H- or a counterion, then $R^2$ is

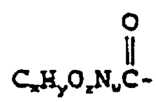

to deliver to the epidermis, as a moisturiser for the skin. the corresponding 2-hydroxyalkanoate having the structure (2):

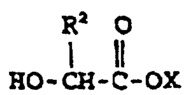 (2)

where

X represents -H or a counterion;

in a composition intended for topical application to human skin, said composition otherwise comprising a cosmetically acceptable vehicle, for the hydroxyalkanoate derivative and a water immiscible oil in an amount exceeding the amount of the hydroxyalkanoate derivative of structure (1) and a penetration enhancer selected from

2-methyl propan-2-ol
Propan-2-ol
Hexan-2,5-diol
POE(2) ethyl ether
Di(2-hydroxypropyl) ether
Pentan-2,4-diol
POE(2) methyl ether
Propan-1-ol
1,4 Dioxane

Propylene glycol dipelargonate
Polyoxypropylene 15 stearyl ether
octyl alcohol
PDE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Dibenxyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl myristate
Isopropyl palmitate
Ethyl laurate
2-ethyl-hexyl pelargonate
Isopropyl isostearate
Butyl laurate
Benzyl benzoate
Butyl benzoate
Hexyl laurate
Ethyl caprate
Ethyl caprylate
Butyl stearate
Benzyl salicylate
2-Pyrrolidone
1-Methyl-2-pyrrolidone
5-Methyl-2-pyrrolidone
1,5-Dimethyl-2-pyrrolidone
1-Ethyl-2-pyrrolidone
Tetrahydrofurfural alcohol
Urea
Diethyl-m-toluamide, and
1-Dodecylazacyloheptan-2-one

When the 2-hydroxyalkanoate is formed in vivo, its counterion may be any ion available in vivo.

The invention also provides a method for delivering to the skin a 2-hydroxy alkanoate having the structure (2):

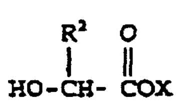

(2)

where

$R^2$ represents H- or $-CH_3$,
X represents H- or a counterion

which comprises the steps of

i) applying topically to the skin a composition comprising the corresponding hydroxy alkanoate derivative having the structure (1):

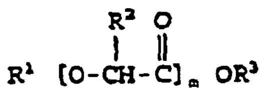

(1)

where $R^1$, $R^3$, x, y, z, w and m have the values as herein before defined, provided that when $R^1$ is H-, then $R^3$ is $C_xH_yO_zN_w$-, or when $R^3$ is H- or a counterion, then $R^1$ is

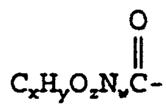

ii) leaving the composition in contact with the skin for at least 10 seconds to permit the hydroxy alkanoate derivative to penetrate through the stratum corneum to reach the lower strata of the epidermis, and

iii) cleaving the hydroxy alkanoate derivative in the epidermis by contact with esterases to provide the 2-hydroxy alkanoate.

The invention also provides a composition suitable for topical application to the skin, which composition comprises:

i) a hydroxy alkanoate derivative having the structure (1) as defined above:

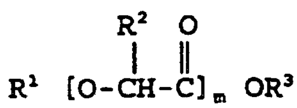

(1)

ii) a cosmetically acceptable vehicle for the hydroxy alkanoate derivative;

the hydroxy alkanoate derivative possessing the ability of penetrating the stratum corneum and being hydrolysed by enzymic cleavage within the epidermis to yield the corresponding hydroxy alkanoate having the structure (2):

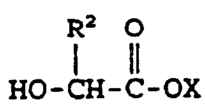

(2)

thereby to moisturise the skin.

In the above formula (1) one preferred possibility is that $R^1$ denotes an acyl group more especially an acyl group

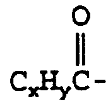

In such a group the value of x is preferably at least 5. A value of at least 9, e.g. 9 to 13 is more preferred.

It is preferred that the vehicle includes water. Certain preferred compositions include water-immiscible oil and/or water-soluble organic solvent such as ethanol. The amount of oil does not usually exceed 50% by weight of the composition, neither does the amount of water-soluble solvent, although in total these non-water materials may provide more than 50% by weight of the composition. Even then, however, the amount of water will normally be not much less than half the composition. Consequently it is preferred that the vehicle contains water in an amount which is at least 43%, or that it conains aqueous ethanol as 50% or more of the composition but with the ethanol providing less than 50% of the composition, it is preferred that water provides at least 43% of the composition.

The invention may be applied in the context of a composition which is applied to skin, especially the face, and either left there or at least not removed immediately.

Penetration into the skin of hydroxyalkanoate derivative from such compositions can occur even when the hydroxyalkanoate is present at rather low concentration.

Such compositions frequently include some oil or oily material or organic solvent which is water soluble.

An alternative is to apply the invention in the context of a washing composition containing surfactant, and intended to be rinsed off after use. With such a composition it will probably be desired to include the hydroxyalkanoate derivative at a higher concentration.

It may also prove convenient to include hydroxyalkanoate derivative at a higher concentration in compositions which contain more than 10% of oil or other involatile organic material such as glycerol.

Consequently it may be stated as a preference within this invention to utilise a concentration of the hydroxyalkanoate derivative which does not exceed 2% by weight of the composition, except when the composition contains at least 10% by weight of a water-immiscible oil, or at least 10% by weight of organic solvent such as glycerol or diglycerol, or at least 10% of a surfactant other than a said acyl derivative of a hydroxyalkanoate.

A low concentration of hydroxyalkanoate may in particular be used when $R^1$ is acyl, i.e.

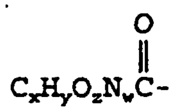

or if $R^3$ contains less than six carbon atoms.

In addition to their benefits in moisturising skin, topical application of the hydroxyalkanoate derivatives can also improve the skin surface in other respects by eliminating or by preventing the development of, or at least slowing, skin surfaces changes. Examples of such changes include, fine lines, wrinkles, blemishes, blotches, nodules, atrophy, pigmented spots, lesions, elastoic changes characterised by leathery, coarse, rough, dry and yellowish skin, photo-damages skin, loss of elasticity and other changes associated with ageing. In summary, the hydroxyalkanoate derivatives can also have marked antiaging benefits to the skin.

The topical application of the hydroxyalkanoate derivatives can also be used for the treatment of spots, pimples and acne, especially for the inhibition of Propionibacterium acnes, which is believed to be a causative organism in the development of acne comedones.

Topical application of the hydroxyalkanoate derivatives can also be used in the treatment of dandruff on the scalp, again in view of the ability of these derivatives to inhibit microorganisms implicated in the development of dandruff.

The preferred hydroxyalkanoate derivatives for inhibiting P. acnes in both acne and dandruff conditions are the $C_{6-12}$ acyl lactylates, especially the $C_8$ acyl species, octanoyl lactylate, which is a powerful inhibitor of P. acnes when applied to acne comedones and to dandruff on the scalp, or as a prophylactic in the treatment of these conditions.

DETAILED DISCLOSURE

The hydroxy alkanoate derivatives

Hydroxy alkanoate derivatives for use in accordance with the invention are chosen from those having the structure (1) as herein defined, and are generally acyl or alkyl derivatives. Acyl derivatives are particularly preferred.

When $R^2$ represents $-CH_3$, the compounds of structure (1) are derivatives of lactic acid. If $R^1$ is acyl, the compounds are frequently termed "lactylates". This name is used for compounds wherein m is one and also compounds wherein m has a higher value, that is to say both

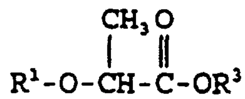

and

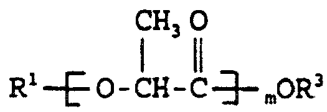

in which m is greater than one.

Examples of acyl derivatives of hydroxy alkanoates include:

(i) those where $R^1$ in structure (1) represents the saturated or unsaturated acyl group:

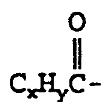

and where

- $R^2$ represents -H or $-CH_3$,
- m is 1 to 5, and
- $R^3$ represents -H, or an alkali metal, alkaline earth metal, ammonium or alkanolammonium counterion.

Specific examples where $R^2$ represents -H are the sodium, potassium, calcium or triethanolammonium salts or free acids of:

n-propionyl glycolate
n-butanoyl glycolate
n-hexanoyl glycolate
n-octanoyl glycolate
n-decanoyl glycolate
n-dodecanoyl glycolate
n-tetradecanoyl glycolate
n-hexadecanoyl glycolate
n-octadecanoyl glycolate
n-linoleoyl glycolate
n-linolenoyl glycolate
γ-linolenoyl glycolate
n-arachidonoyl glycolate
columbinoyl glycolate, and

the corresponding di-, tri-, tetra- and pentaglycolates.

Specific examples where $R^2$ represents $-CH_3$ are the sodium, potassium, calcium, ammonium or triethanolammonium salts or free acids of:

n-propionyl lactylate
n-butanoyl lactylate

n-hexanoyl lactylate
n-octanoyl lactylate
n-decanoyl lactylate
n-dodecanoyl lactylate
n-tetradecanoyl lactylate
n-hexadecanoyl lactylate
n-octadecanoyl lactylate
n-linoleoyl lactylate
n-linolenoyl lactylate
γ-linolenoyl lactylate
n-arachidonyl lactylate, and
columbinoyl lactylate,

both as the monolactylates and as the corresponding di-, tri-, tetra- and pentalactylates.

(ii) those where $R^1$ in structure (1) represents the branched chain acyl group:

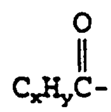

where

$R^2$ represents -H or $CH_3$,
$C_xH_y$- represents a branched chain alkyl group
m is 1 to 5, and
$R^3$ represents -H, or an alkali metal, alkaline earth metal, ammonium or alkanolammonium counterion.

Specific examples where $R^2$ represents -H are the sodium, potassium, calcium or triethanolammonium salts or free acids of:

iso-butanoyl glycolate
iso-hexanoyl glycolate
iso-octadecanoyl glycolate, and
the corresponding di-, tri-, tetra- and pentaglycolates.

Specific examples where $R^2$ represents $-CH_3$ are the sodium, potassium, calcium, ammonium or triethanolammonium salts or free acids of:

iso-butanoyl lactylate
iso-hexanoyl lactylate
iso-octanoyl lactylate, and
iso-octadecanoyl lactylate,

both as monolacylates and as the corresponding di-, tri-, tetra- and pentalactylates.

(iii) those where $R^1$ in structure (1) represents the saturated or unsaturated acyl or aryl group:

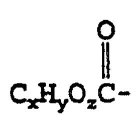

where

$R^2$ represents -H or -$CH_3$
m is 1 to 5, and
$R^3$ is -H, or an alkali metal, alkaline earth metal, ammonium or alkanolammonium counterion.

Specific examples where $R^2$ represents -H are the sodium, potassium, calcium, ammonium or tri ethanolammonium salts or free acids of:

ethyl glycoloyl glycolate
leucoyl glycolate
mandeloyl glycolate, and

the corresponding di-, tri-, tetra- and pentaglycolates.

Specific examples where $R^2$ represents -$CH_3$ are the sodium, potassium, calcium, ammonium or triethanolammonium salts or free acids of:

12-hydroxy octadecanoyl lactylate
ethyl glycoloyl lactylate
leucoyl lactylate, and
mandeloyl lactylate,

both as monolactylates and as the corresponding di-, tri-, tetra- and pentalactylates.

(iv) those where $R^1$ in structure (1) represents the saturated or unsaturated acyl group.

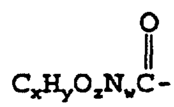

where

$R^2$ represents -H or -$CH_3$
m is 1 to 5, and
$R^3$ represents -H, or an alkali metal, alkaline earth metal, ammonium or alkanolammonium counterion.

Specific examples where $R^2$ represents -H are the sodium, potassium, calcium, ammonium or triethanolammonium salts or free acids of:

cocoamidopropanoyl glycolate
pyroglutamoyl glycolate
cholesteroyl glycolate
"ceramidoyl glycolate", and

the corresponding di-, tri-, tetra- and pentaglycolates.

Specific examples where $R^2$ represents -$CH_3$ are the sodium, potassium, calcium, ammonium or triethanolammonium salts or free acids of:

cocoamidopropanoyl lactylate
pyroglutamoyl lactylate
cholesteroyl lactylate, and
"ceramidoyl lactylate",

both as monolactylates and as the corresponding di-, tri-, tetra- and pentalactylates.

Examples of alkyl hydroxyalkanoates that conform with structure (1) are those where:

$R^1$ in Structure (1) represents H-,

$R^2$ represents H- or $-CH_3$,
$R^3$ represents $C_xH_y$-, and
m is 1.

Specific examples are:

methyl glycate
n-butyl glycate
methyl lactate and
n-butyl lactate

Preferred examples in which x is at least 5 or 6 are:

n-hexyl glycate
n-octyl glycate
n-decyl glycate
n-dodecyl glycate
n-octadecyl glycate
n-hexyl lactate
n-octyl lactate
n-decyl lactate
n-dodecyl lactate
n-tetradecyl lactate
n-hexadecyl lactate
n-octadecyl lactate
2-octyldecyl lactate
octyl dodecyl lactate, and
palmitoyl glyceryl lactate.

It is not intended that the above examples form an exhaustive list of acyl derivatives of hydroxy alkanoates and alkyl hydroxy alkanoates, as there are many more compounds which comply with the structure (1), that can be used in accordance with the invention.

The amount of hydroxyalkanoate derivative to be employed in accordance with the invention as an effective amount, will normally be from 0.001 to 50%, preferably at least 0.05%, for example from 0.05 to 30%, and most preferably at least 0.1%, for example from 0.1 to 20% by weight of the composition.

As will be explained and illustrated later in the specification, the composition for use in accordance with the invention can either be employed as a "leave-on" product, in which case it is intended to be applied to the skin and left in place, or it can be employed as a "rinse-off" or "wipe-off" product, usually for cleansing the skin, in which case it will normally be rinsed or wiped from the skin shortly after application. Thus for "leave-on" products, the most preferred amount of hydroxyalkanoate derivative will be up to about 5% more preferably from 0.1 to 2%, whereas, for "rinse-off" or - "wipe-off" products, the corresponding preferred amount will be from 0.1 to 20% or more, for example up to 30%, in both instances expressed in terms of percentage by weight of the composition. For wipe-off products the preferred amount may be up to about 5%.

Cosmetically Acceptable Vehicles

The hydroxyalkanoate derivatives, as herein described, will in use normally be applied to human skin in the form of a composition that also comprises a cosmetically acceptable vehicle, that is intended to facilitate the distribution of the hydroxyalkanoate derivative on and over the skin surface at an appropriate concentration.

The composition can thus be solid, semi-solid or liquid in nature, dependent upon the choice of vehicle. The vehicle itself can be inert or it can possess beneficial physiological properties of its own.

The selection of a vehicle for this purpose presents a wide range of possibilities depending on the required product form of the composition. Suitable vehicles can be classified as described hereinafter.

Vehicles are therefore substances that can act as diluents, dispersants, or solvents for the hydroxy alkanoate derivative which ensures that it can be applied to and distributed evenly over the skin at an appropriate concentration. The vehicle is preferably one which can aid penetration of the hydroxy alkanoate derivative deep into the epidermis, to enable it more readily to influence the condition of the skin.

Compositions according to the invention can include water as a vehicle, and/or at least one cosmetically

acceptable vehicle other than water.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, squalane, squalene, butyl myristate, isostearic acid, palmitic acid, isopropyl linoleate, decyl oleate, myristyl myristate;

Propellants for a pressurized aerosol such as propane, butane, isobutane, dimethyl ether, carbon dioxide, nitrous oxide;

Solvents, such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide, tetrahydrofuran;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate, ethylene glycol distearate;

The cosmetically acceptable vehicle will usually form from 10 to 99.99%, preferably from 10 to 99% and most preferably from 50 to 99% by weight of the composition, and can, in the absence of other cosmetic adjuncts, form the balance of the composition.

## OPTIONAL SKIN BENEFIT MATERIALS AND COSMETIC ADJUNCTS

### Penetration Enhancer

The composition for use according to the invention can also optionally comprise a penetration enhancer which can potentiate the benefit of the hydroxy alkanoate derivative by improving its delivery through the stratum corneum to its site of action deep in the epidermis.

The penetration enhancer can accordingly function in a variety of ways. It can for example, improve the distribution of the hydroxy alkanoate derivative on the skin surface, or it can increase its partition into the stratum corneum and beyond from the composition when applied topically, so aiding its passage to a lower level within the epidermis. Other mechanisms enhancing the benefit of the hydroxy alkanoate derivative may also be involved.

Examples of penetration enhancers include:

2-methyl propan-2-ol
Propan-2-ol
Hexan-2,5-diol
POE(2) ethyl ether
Di(2-hydroxypropyl) ether
Pentan-2,4-diol
POE(2) methyl ether
Propan-1-ol
1,4 Dioxane
Butan-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate

Dibutyl sebacate
Diethyl sebacate
Dimethyl sebacate
Dioctyl sebacate
Dibutyl suberate
Dioctyl azelate
Dibenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl myristate
Isopropyl palmitate
Ethyl laurate
2-ethyl-hexyl pelargonate
Isopropyl isostearate
Butyl laurate
Benzyl benzoate
Butyl benzoate
Hexyl laurate
Ethyl caprate
Ethyl caprylate
Butyl stearate
Benzyl salicylate
2-Pyrrolidone
1-Methyl-2-pyrrolidone
5-Methyl-2-pyrrolidone
1,5-Dimethyl-2-pyrrolidone
1-Ethyl-2-pyrrolidone
Sugar esters
Tetrahydrofurfural alcohol
Urea
Diethyl-m-toluamide, and
1-Dodecylazacyloheptan-2-one

The amount of penetration enhancer, when employed in accordance with the invention, will normally be from 0.1 to 50%, preferably from 0.5 to 25% and most preferably from 0.5 to 10% by weight of the composition.

Emulsions and Oil Materials

A composition according to the invention can optionally contain one or more oils or other materials which have the properties of an oil and are immiscible with water.

A composition according to the invention may be formulated as an emulsion having both aqueous and oil phases. This applies particularly to leave-on products which are frequently formulated as emulsions. In an emulsion an oil or oily material will normally be present, together with an emulsifier to provide either a water-in-oil emulsion or an oil-in-water emulsion, depending largely on the average hydrophilic-lipophilic balance (HLB) of the emulsifier employed.

Examples of suitable oils include mineral oil and vegetable oils, silicone oils and oily materials, such as those already proposed herein as emollients.

The quantity of oil, if present, is often at least 5% by weight, but often remains a minority of the composition, in other words 5 to 50% by weight. Preferred are at least 10% and/or not more than 30% by weight.

The oil or oily material, when present for the purposes for forming an emulsion,.may form up to 90%, e.g.

from 10 to 80% by volume of the composition.

Emulsifier

The composition according to the invention can also optionally comprise one or more emulsifiers the choice of which will normally determine whether a water-in-oil or and oil-in-water emulsion is formed. Duplex emulsions, i.e. water-in-oil-in water or oil-in-water-in-oil can also be formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set below in Table 1 in which the chemical name of the emulsifiers is given together with an example of a trade name as commercially available, and the average HLB value.

TABLE 1

| Chemical Name of Emulsifier | Trade Name | HLB Value |
|---|---|---|
| Sorbitan trioleate | Arlacel 85 | 1.8 |
| Sorbitan tristearate | Span 65 | 2.1 |
| Glycerol monooleate | Aldo MD | 2.7 |
| Glycerol monostearate | Atmul 84S | 2.8 |
| Glycerol monolaurate | Aldo MC | 3.3 |
| Sorbitan sesquioleate | Arlacel 83 | 3.7 |
| Sorbitan monooleate | Arlacel 80 | 4.3 |
| Sorbitan monostearate | Span 60 | 4.7 |
| Cetyl dimethicone copolyol | Abil EM-90 | 4 to 6 |
| Poloxyethylene (2) stearyl ether | Brij 72 | 4.9 |
| Poloxyethylene sorbitol beeswax derivative | G-1702 | 5 |
| PEG 200 dilaurate | Emerest 2622 | 6.3 |
| Sorbitan monopalmitate | Arlacel 40 | 6.7 |
| Polyoxyethylene (3.5) nonyl phenol | Emulgen 903 | 7.8 |
| PEG 200 monostearate | Tegester PEG 200 MS | 8.5 |
| Sorbitan monolaurate | Arlacel 200 | 8.6 |
| PEG 400 dioleate | Tegester PEG 400-DO | 8.8 |
| Polyoxyethylene (5) monostearate | Ethofat 60-16 | 9.0 |
| Polyoxyethylene (4) sorbitan monostearate | Tween 61 | 9.6 |
| Polyoxyethylene (4) lauryl ether | Brij 30 | 9.7 |
| Dimethicone copolyol | Abil B 8842 | >10 |
| Polyoxyethylene (5) sorbitan monooleate | Tween 81 | 10.0 |
| PEG 300 monooleate | Neutronyx 834 | 10.4 |
| Polyoxyethylene (20) sorbitan tristearate | Tween 65 | 10.5 |
| Polyoxyethylene (20) sorbitan trioleate | Tween 85 | 11.0 |
| Polyoxyethylene (8) monostearate | Myrj 45 | 11.1 |
| PEG 400 monooleate | Emerest 2646 | 11.7 |
| PEG 400 monostearate | Tegester PEG 400 | 11.9 |
| Polyoxyethylene 10 monooleate | Ethofat 0/20 | 12.2 |
| Polyoxyethylene (10) stearyl ether | Brij 76 | 12.4 |
| Polyoxyethylene (10) cetyl ether | Brij 56 | 12.9 |
| Polyoxyethylene (9.3) octyl phenol | Triton X-100 | 13.0 |
| Polyoxyethylene (4) sorbitan monolaurate | Tween 21 | 13.3 |
| PEG 600 monooleate | Emerest 2660 | 13.7 |
| PEG 1000 dilaurate | Kessco | 13.9 |
| Polyoxyethylene sorbitol lanolin derivative | G-1441 | 14.0 |
| Polyoxyethylene (12) lauryl ether | Ethosperse LA-12 | 14.4 |
| PEG 1500 dioleate | Pegosperse 1500 | 14.6 |

TABLE 1 (continued)

| Chemical Name of Emulsifier | Trade Name | HLB Value |
|---|---|---|
| Polyoxyethylene (14) laurate | Arosurf HFL-714 | 14.8 |
| Polyoxyethylene (20) sorbitan monostearate | Tween 60 | 14.9 |
| Polyoxyethylene 20 sorbitan monooleate | Tween 80 | 15.0 |
| Polyoxyethylene (20) stearate | Myrj 49 | 15.0 |
| Polyoxyethylene (20) stearyl ether | Brij 78 | 15.3 |
| Polyoxyethylene (20) sorbitan monopalmitate | Tween 40 | 15.6 |
| Polyoxyethylene (20) cetyl ether | Brij 58 | 15.7 |
| Triethanolamine stearate | | >16 |
| Polyoxyethylene (25) oxypropylene monostearate | G-2162 | 16.0 |
| Polyoxyethylene (20) sorbitol monolaurate | Tween 20 | 16.7 |
| Polyoxyethylene (23) lauryl ether | Brij 35 | 16.9 |
| Polyoxyethylene (50) monostearate | Myrj 53 | 17.9 |
| PEG 4000 monostearate | Pegosperse 4000 MS | 18.7 |

The foregoing list of emulsifiers is not intended to be limiting and merely exemplifies selected emulsifiers which are suitable for use in accordance with the invention.

It is to be understood that two or more emulsifiers can be employed if desired.

The amount of emulsifier or mixtures thereof, to be incorporated in the composition of the invention, when appropriate is from 1 to 50%, preferably from 2 to 20% and most preferably from 2 to 10% by weight of the composition.

Retinoids

The composition for use according to the invention optionally can also comprise a retinoid, such as retinoic acid or retinol (Vitamin A) and/or derivative thereof, further to enhance the benefits to skin.

In addition to retinol itself, examples of derivatives of retinol include:

Retinyl acetate
Retinyl butyrate
Retinyl propionate
Retinyl octanoate
Retinyl laurate
Retinyl palmitate
Retinyl oleate
Retinyl linoleate, and
Retinyl linolenate.

The amount of retinoid, when present in the composition according to the invention is from 0.01 to 10% and preferably 0.1 to 5% by weight of the composition.

Tocopherol and Tocopheryl Esters

The composition for use according to the invention optionally can also comprise a tocopherol (vitamin E group), as an antioxidant for the composition, and to limit oxidative damage to skin. The vitamin E group comprises $\alpha$-tocopherol, $\beta$-tocopherol, $\gamma$-tocopherol and $\delta$-tocopherol. The composition according to the invention optionally can also comprise a tocopheryl ester, such as tocopheryl acetate.

The amount of a tocopherol, or ester thereof, when present in the composition according to the invention, is from 0.0001 to 20%, preferably from 0.0001 to 10% by weight of the composition.

Water

The composition of the invention can also comprise water, usually up to 90%, preferably from 5 to 80% by volume.

Water can function as the cosmetically acceptable vehicle.

Preferred is that the vehicle contains water, or aqueous ethanol, in an amount which is a majority of the composition, that is to say over 50%. Even when ethanol is included, the quantity of water will generally be substantial, amounting to a majority of the composition or nearly so, such as at least 40% of the composition.

Surfactants

An acyl or alkyl derivative of hydroxyalkanoate can function as a surfactant and can then be utilised as such in a composition for use in accordance with this invention which is formulated as a cleaning product. In this event it may be present in a substantial amount, notably exceeding 5% or 10% by weight of the composition.

A composition for use in accordance with the invention, especially if formulated as a cleansing product, may comprise one or more surfactants, in addition to the hydroxy alkanoate derivatives, which are cosmetically acceptable and suitable for topical application to the skin. However, the amount of any surfactant other than the hydroxyalkanoate derivative may be small, especially for leave-on or wipe-off products, which may well contain only a single figure percentage of material with surfactant properties, notably of nonionic surfactant without any anionic surfactant other than the hydroxyalkanoate derivative.

Examples of other suitable skin cleansing surfactants are now given.

Anionic surfactant

The composition of the invention can comprise soap or a non-soap anionic surfactant which is preferably chosen from alkyl sulphate, alkyl ether sulphate, alkyl sulphonate, alkyl aryl sulphonate, olefin sulphonate, acyl sarcosinate, acyl tauride, acyl isethionate, nonoalkyl sulphosuccinate, dialkylsulphosuccinate, N-acylated $\alpha$-amino acid, alkyl carboxylate, monoalkyl phosphate and dialkyl phosphate.

Specific examples of anionic surfactants include:

alkyl sulphates, such as sodium lauryl sulphate [eg. EMPICOL CX available from Albright & Wilson], and triethanolaminde lauryl sulphate [e.g. EMPICOL TL40/T, available from Albright & Wilson].

alkylether sulphates, such as sodium lauryl ether sulphate [eg. EMPICOL ESB70, available from Albright & Wilson].

alkyl sulphonates, such as sodium alkane ($C_{13-18}$) sulphonate [eg. HOSTAPUR SAS 30, available from Hoechst].

alkylaryl sulphonates, such as sodium alkyl benzene sulphonate [eg. TEEPOL CM44, available from Shell].

olefin sulphonates, such as sodium olefin sulphonate ($C_{5-18}$) [eg. HOSTAPUR OS, available from Hoechst].

acyl sarcosinates, having the structure: (51)

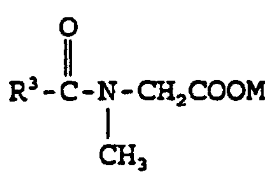

(51)

where $R^3$ is chosen from $C_{6-14}$, alkyl, and

M is a counterion chosen from alkali metals, ammonium and substituted ammonium such as alkanolammonium.

An example of an acyl sarcosinate having the structure (51), is sodium lauryl sarcosinate [eg. HAMPOSYL L-95, available from Grace].

acyl taurides, having the structure (52):

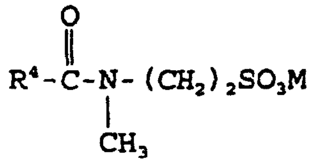

(52)

where $R^4$ is chosen from $C_{8-18}$ alkyl

An example of an acyl tauride having the structure (52) is coconut methyl taurine [eg. FENOPEN TC 42, available from International Specialty Products].

acyl isethionates, having the structure (53):

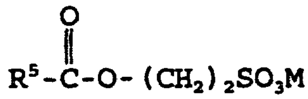

(53)

where $R^5$ is chosen from $C_{8-18}$ alkyl.

An example of an acyl isethionate having the structure (53) is sodium acyl isethionate [eg. JORDAPON C1, available from Jordon].

monoalkyl sulphosuccinates, having the structure (54):

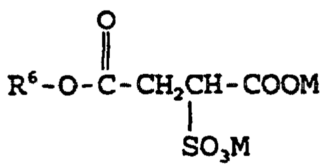

(54)

where $R^6$ is chosen from $C_{10-20}$ alkyl.

Examples of monoalkyl sulphosuccinates having the structure (54) include:

sodium lauryl sulphosuccinate [eg. EMPICOL SLL, available from Albright & Wilson].

magnesium alkyl sulphosuccinate [eg. ELFANOL 616 Mg, available from AKZO].

sodium lauryl ethoxysulphosuccinate [eg. EMPICOL SDD, available from Albright & Wilson].

coconut monoethanolamide ethoxysulphosuccinate [eg. EMPICOL SGG].

disodium lauryl polyglycolether sulphosuccinate (eg. SURTAGENE S30, available from CHEM-Y].

polyethyleneglycol sulphosuccinate [eg. REWOPOL SBFA 30, available from REWO].

dialkyl sulphosuccinates, having the structure (55):

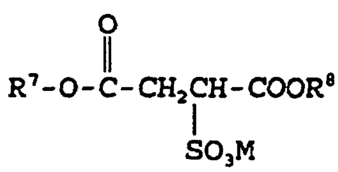

(55)

where $R^7$ and $R^8$ are the same or different, and are chosen from $C_{6-14}$ alkyl.

An example of a dialkyl sulphosuccinate having the structure (55) is sodium dilauryl sulphosuccinate [eg. EMCOL 4500, available from Witco].

N-acylated α-amino acids, such as sodium lauroyl glutamate [eg. ACYLGLUTAMATE LS-11, available from Ajinomoto Co. Inc].

alkyl ether carboxylates, such as $C_{12-14}O(EO)_4OCH_2CO_2Na$ [eg. AKYPO RLM 38, available from Akzo].

monoalkyl phosphates and dialkyl phosphates, such as dioctyl phosphate.

Cationic surfactant

The composition of the invention can also comprise a cationic surfactant. Suitable cationic surfactants are those with the structure (57):

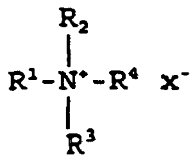 (57)

where

$R^1$, $R^2$, $R^3$ and $R^4$ each represents alkyl or aryl groups,
and
X represents al halogen counterion.

Preferred cationic surfactants in accordance with structure (57) include:
Hexadecyl trimethyl ammonium chloride, such as Arquad 16, available from Akzo.
Dihydrogenated tallow dimethyl ammonium chloride, such as Arquad 2HT, available from Akzo.
Dodecyl benzyl dimethyl ammonium bromide, such as Amoxyl BR 1244, available from Seppic.
Cocoamidopropyl trimethyl ammonium chloride, such as Empigen CSC, available from Albright & Wilson.

Amphoteric surfactant

The composition of the invention can also comprise an amphoteric surfactant. Suitable amphoteric surfactants are derivatives of aliphatic quaternary ammonium, phosphonium and sulphonium compounds, wherein the aliphatic radicals contain from 8 to 18 carbon atoms, and may be straight chain or branched, and further contain an anionic water-solubilising group, such as carboxyl, sulphonate, sulphate, phosphate or phosphonate.
Preferred amphoteric surfactants include:
Alkyl betaines. having the structure (58):

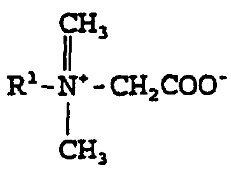 (58)

where $R^1$ is $C_{1-16}$ alkyl.
An example of an alkyl betaine having the structure (58) is lauryldimethyl betaine [eg. EMPIGEN BB, available from Albright & Wilson].
Alkylamidopropyl betaines, having the structure (59):

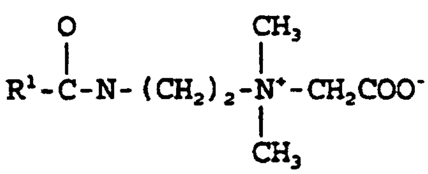 (59)

An example of an alkylamidopropyl betaine having the structure (59) is cocamidopropyl betaine [eg. TEGOBETAIN L7, available from Goldschmidt).
Alkylamphoglycinates or Alkylamphopropionates having the structure (60):

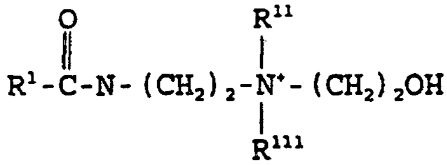 (60)

where $R^{11}$ is chosen from H, $CH_2COO$- and $(CH_2)_2COO^-$, and $R^{111}$ is chosen from $CH_2COO$- and $(CH_2)_2COO^-$

Suitable examples of compounds (60) are cocoamphoglycinate (available from International Specialty Products), and cocoamphopropionate.

Sultaines, having the structure (61):

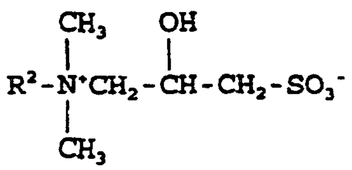 (61)

where $R^2$ is chosen from $C_{12-16}$ alkyl alkylamido groups.

An example of a sultaine having the structure (61) is cocamidopropylhydroxysultaine (eg. CYCLOTERIC BET-CS, available from Alcolac).

The most preferred amphoteric surfactant are lauryl dimethyl betaine and cocamidopropyl betaine.

Such amphoteric surfactants can contribute to the foaming of the skin cleansing composition, while ameliorating the harshness of the anionic surfactant.

Nonionic surfactant

The composition of the invention can also comprise alkoxylated or glycosidic nonionic surfactant having an HLB of 8 or more. Above this value nonionics generally form clear isotropic solutions in combination with the other surfactants in the ranges defined above. Preferred nonionic surfactants are polyoxyethylene alkyl esters and polyoxyethylene alkyl ethers and alkyl polyglycosides.

A suitable example of a polyoxyethylene alkyl ester is that having the CTFA designation Polysorbate 80 which is a mixture of oleate esters of sorbitol and sorbitol anhydrides, condensed with approximately 20 moles of ethylene oxide. Also suitable is Polysorbate 20 which is a mixture of laurate esters or sorbitol and sorbitol anhydrides condensed with approximately 20 moles of ethylene oxide.

Polysorbate 80 and Polysorbate 20 are available commercially as TWEEN 80 and TWEEN 20 respectively, from ICI Americas.

Also suitable for use in the compositions of the invention is the polyethylene glycol ether of $C_{9-11}$ alcohol with an average of 8 ethoxy units, which is available commercially as NONIDET LE-8T or as SYNPERONIC 91-8T, and the polyethylene glycol ether of $C_{12-15}$ alcohol with an average of 9 ethoxy units which is available commercially as DOBANOL 25-9.

Particularly useful alkyl polyglycosides include the glycosides of glucose or glucose oligomers where the alkyl chain can be $C_{8-16}$ and the average number of glucose units is 1 to 2. A suitable example is ORAMIX NS 10 which is the glucoside of $C_{10-12}$ fatty alcohol with an average of about 1.5 glucose units.

Also suitable for use in compositions of the invention are high molecular weight silicone surfactants, such as a high molecular weight polymer of dimethyl polysiloxane with polyoxyethylene and/or polyoxypropylene side chains having a molecular weight from 10,000 to 50,000.

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C Formulation Aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the composition.

The amount of other surfactant that can be present in the composition for use in accordance with the invention is generally not over 30%, preferably from 1 to 20% by weight of the composition.

Other Cosmetic Adjuncts

Examples of other cosmetic adjuncts which can optionally be employed in the composition for use according to the invention include preservatives, such as para-hydroxy benzoate esters; antioxidants, such as butyl hydroxy toluene; humectants, such as glycerol, sorbitol, 2-pyrrolidone-5-carboxylate, dibutylphthalate, gelatin, polyethylene, glycol, preferably PEG 200-600; buffers, such as lactic acid together with a base such as triethanolamine or sodium hydroxide; ceramides of synthetic, animal or plant origin; pseudoceramides; phospholipids; vitamins, such as 1,25 dihydroxy cholecalciferol; waxes, such as beeswax, ozokerite wax, paraffin wax, plant extracts, such as Aloe vera, cornflower, witch hazel, elderflower, cucumber, thickeners; activity enhancers; colourants; perfumes; and sunscreen materials such as ultrafine titanium dioxide and organic sunscreens such as p-aminobenzoic acid and esters thereof, ethylhexyl p-methoxycinnamate, 2-ethoxyethyl p-methoxycinnamate and butyl methoxydibenzoylmethane, and mixtures thereof.

In a further preferred composition, the hydroxyalkanoate derivative is combined with ceramides, pseudoceramides, polyol fatty acid polyesters, sterols, particularly cholesterol, galactosyldiacyl-glycerols, glycosphingolipids, fatty acids and esters thereof and mixtures thereof and other ingredients, such as mevalonic acid, hexadecylsuccinic acid monobehenyl ester ethoxylate (7.3 EO) and/or derivatives thereof to produce a liposomal dispersion.

A further preferred composition may also contain in combination with the hydroxy alkanoate derivative and optional additional ingredients disclosed above, an organic acid component chosen from hydroxy alkanoic acids, such as alpha, beta and omega hydroxyacids, especially glycolic acid, lactic acid, citric acid, tartaric acid, tartronic acid and 2-hydroxyoctanoic acid, and keto carboxylic acids, esters thereof and mixtures thereof.

Cosmetic adjuncts can form the balance of the composition. pH

The composition of the invention will normally have a pH value of from 4 to 9, preferably from 4.5 to 8.5. The pH can be adjusted as necessary by the addition of an alkali or acid as a pH adjustant, and/or by the addition of a buffer, such as a citrate buffer or a phosphate buffer.

PRESERVATION OF THE COMPOSITION

The composition for use in accordance with the invention is preferably preserved against attack by bacteria, moulds and fungi and other microbial influences, in such a manner that it will enjoy an extended shelf life following manufacture and prior to sale and use. Ideally the composition will have an indefinite shelf life.

Examples of the methods that can be employed to achieve preservation of the composition, include the following:

(i) Sterilisation

The composition according to the invention can be preserved by sterilisation to remove or kill substantially all viable microbial contaminants. This can be achieved for example by irradiation using a lethal dose of gamma rays, by heat sterilisation or by ultrafiltration using techniques that are well established in the pharmaceutical industry.

(ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of-p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

(iii) Water activity depressants

The composition according to the invention can also be preserved by the inclusion of a water activity depressant such as glycerol, propylene glycol, sorbitol, sugars and salts, for examples alkali metal halides, sulphates and

carboxylates. When employing a water activity depressant, sufficient should be incorporated in the composition according to the invention to reduce the water activity ($\alpha_w$) from 1 to < 0.9, preferably to < 0.85 and most preferably < 0.8, the lowest of these values being that at which yeasts, moulds and fungi will not proliferate.

## PROCESS

The invention also provides a process for preparing a composition according to the invention which comprises the steps of mixing an effective amount of an hydroxy alkanoate derivative, as herein defined, together with a cosmetically acceptable carrier for the derivative.

## Product Form and Container

The composition of the invention can be formulated as liquids, for example as a lotion, shampoo, milk or cream for use in conjunction with an applicator such as roll-ball applicator, or a spray device such as an aerosol can containing propellant, or a container fitted with a pump to disperse the liquid product. Alternatively, the compositions of the invention can be solid or semi-solid, for example sticks, creams or gels, for use in conjunction with a suitable applicator or simply a tube, bottle or lidded jar, or as a liquid-impregnated fabric such as a tissue wipe.

The invention accordingly also provides a closed container containing a composition as herein defined.

## USE OF THE COMPOSITION

The acyl (and/or alkyl) derivatives of hydroxyalkanoate, when applied to the stratum corneum, will penetrate into the epidermis or cutaneous appendages, such as eccrine, apocrine and sebaceous glands, where hydrolysis by endogenous skin or microbial esterases will cleave the molecule to release in situ the corresponding hydroxy alkanoate, together with the fatty acid or fatty alcohol that forms the residue of the applied hydroxy alkanoate derivative.

As stated earlier, the composition as applied to the skin surface can either be a "leave-on" product or a "rinse-off" or "wipe-off" product. When the composition is a leave-on product, such as a cream or lotion, it can be applied to the skin from a suitable container and spread over a desired area of skin, such as the face and neck, shoulders, arms and legs, and then rubbed in using the hands and fingers, or using an applicator. The hydroxyalkanoate derivative being lipophilic in nature, will penetrate into and through the stratum corneum, by virtue of the lipid materials that normally reside in that region close to the skin surface. On reaching the strata below the superficial stratum corneum cells, or penetration into the cutaneous appendages, esterases are encountered that hydrolyse the hydroxy alkanoate derivative to release the desired hydroxyalkanoate.

Repeated application of such leave-on products, for example at night and in the morning on a daily basis, will provide a continuous supply of hydroxyalkanoate to the epidermis, which will thereby benefit by its improving ability to retain moisture i.e. to become moisturised. Other attributes of the skin will also improve, particularly elasticity, disappearance of fine lines and wrinkles, and the skin will altogether become more healthy with improved tone and condition.

When the composition for use in accordance with the invention is a "rinse-off" product, it will generally function as a skin cleanser. A suitable amount, for example 5 to 10 ml of the skin cleanser comprising the hydroxy alkanoate derivative, which can itself have surfactant properties, but is preferably accompanied by a co-surfactant, is applied to the skin and formed into a lather in the presence of water. After cleansing the skin, surplus product is generally rinsed from the skin and the skin is then dried. The rinse-off product can also be used for washing the hair or for cleansing the entire body surface, for example in the shower.

Although when using a rinse-off cleansing product, it would be expected that little or no product would remain on the skin, we have surprisingly found that an amount of the hydroxy alkanoate derivative is retained by the skin. This enables the skin to retain moisture to a greater extent than with other "rinse-off" products.

For this reason a hydroxyalkanoate derivative in accordance with this invention might be included as part of a composition for washing dishes by hand, thereby to enhance its mildness.

When the composition is a wipe-off product, it will also generally function as a skin cleanser, especially for removing make-up. A suitable amount, for example 0.5 to 5ml of the skin cleanser comprising the hydroxy alkanoate derivative can be applied to the skin, particularly where make-up is to be removed, and rubbed-in. The area of treated skin can then be wiped with a cloth, tissue or with cotton wool to remove surplus of the composition together with make-up that has been loosened from the skin.

Particularly preferred examples of both "rinse-off" and "leave-on" products in accordance with the invention are those that are used as part of the normal shaving process, namely as a pre-shave product, or as an after-shave product. Both products in use are intended to moisturise or otherwise condition the skin, prior to or after shaving, so as to enhance the shaving process, and this can be achieved by delivery to the epidermis of an hydroxy alkanoate, in the manner described hereinbefore.

Shaving treatment products such as these can be employed either by men to remove unwanted facial hair or beard, or by women to remove unwanted hair from other parts of the body surface, particularly the legs. Wherever such products are used, they possess the ability to enhance the moisturisation of the skin, so improving its condition after the shaving process.

Because men's shaving products are used regularly, they can serve to provide regularly delivery of hydroxyalkanoate to the skin.

According to a particularly preferred embodiment of this aspect of the invention, after-shave products are formulated with iso-stearoyl lactylate, and are further characterised by containing, as a cosmetically acceptable vehicle for the acyl lactylate, more than about 85% by weight of ethanol and water. Additionally, such shaving products can contain other cosmetic adjuncts such as glycerine, perfume, perfume solubilisers to promote product clarity, vitamins and derivatives thereof such as vitamin E acetate and Vitamin A palmitate, thickeners such as Carbopol 980, triethanolamine and other adjuncts as conventionally used in pre- or after-shave products.

These products upon application to the skin, especially the face, enhance the moisturisation of the skin by delivery to the stratum corneum and to the lower strata of the epidermis of lactic acid and lactate ions.

## EXAMPLES

### Example 1. Demonstrating Delivery of Lactate to the Epidermis

When an acyl lactylate is applied to the surface of the skin, it migrates by adsorption to a greater or lesser degree through the stratum corneum, dependent upon its lipophylicity. On contact with skin and/or microbial esterases, the acyl lactylate molecule is cleaved and lactic acid/lactate is released within the stratum corneum, or cutaneous appendage and deeper in the epidermis.

In contrast, when lactic acid is applied to the skin surface, very little of it penetrates.

The penetration of acyl lactylate and lactic acid in this manner can be determined by serial tape stripping and by biochemical assay of free lactate from skin cells adhering to the tape.

The methodology employed using pig skin is as follows:

### Measurement of Lactate in Stratum Corneum

The tape employed in this test is Desquame tape available from Diastron. Following topical application to skin of acyl lactylate, pieces of this tape are applied to the skin and then removed, and skin cells are assayed for lactate using Sigma 735-10 lactate assay kit. Thus, by repeated stripping of the same area of skin the degree of penetration of the topically applied acyl lactylate and its cleavage by epidermal esterases can be determined.

### Penetration of Acyl Lactylates, the Effect of Chain Length

10% by weight solutions of sodium lactate (control) and of five different sodium acyl lactylates having from $C_8$ to $C_{18}$ acyl chains, were prepared in pH 7.0 Sorenson's phosphate buffer, 5 mM. Seven pieces of full thickness skin, taken from pig ear, were cut to 4 x 4cm and pinned out. 100 µl aliquots of the 6 test solutions were applied to the entire surface of six pieces of skin, the remaining piece of skin was used as a no-treatment control. The skin was then allowed to stand at room temperature for four hours. Ten sequential tape strips using Desquame tape were then taken from the centre of each piece of skin, ensuring that the tapes were taken from the same site each time.

Each tape was placed in a labelled 1ml tube and 800µl of 5.00mM phosphate buffer, pH 7.00 was added to each tube. The tubes were then cycled from -20 to 20°C three times, with a minimum of 30 mins at each temperature, and then placed in an ultrasound bath for 30 mins. A bottle of Sigma 735-10 lactate reagent was then diluted with 5.00ml analar water, and 200µl aliquots were then transferred to each tube. The tubes were then allowed to stand at room temperature for a further 15 minutes. The buffer reagent mixture from each tube was then transferred to microcuvettes and the absorbtion of the solutions measured at 540nm using a spectrophotometer.

In the following table, tape 1 refers to the first tape strip taken from each site, and the values given are absorption units which are directly proportional to the concentration of L-lactic acid present on each tape.

These tabulated results are also reproduced in figure 1 which shows a plot of absolute units of L-lactic acid as determined by the Sigma assay (abscissa or x-axis) against the number of tape strips which is indicative of depth below skin surface (ordinate or y-axis).

### Conclusions

It can be concluded from these results that each of the five selected sodium acyl lactylates penetrates into

the stratum corneum, where enzymic cleavage to release lactic acid/lactate occurs, whereas with sodium lactate applied topically, virtually no penetration occurs, as the values of lactic acid/lactate recorded are almost identical with those of the untreated control skin sample.

Similar results are obtainable when using acyl glycolates.

Tape No.

| | 1 | 2 | 3 | 4 | 5 | 6 | 7 | 8 | 9 | 10 |
|---|---|---|---|---|---|---|---|---|---|---|
| A | 0.041 | 0.051 | 0.031 | 0.030 | 0.029 | 0.030 | 0.039 | 0.035 | 0.034 | 0.035 |
| B | 0.276 | 0.198 | 0.191 | 0.171 | 0.105 | 0.087 | 0.091 | 0.067 | 0.096 | 0.078 |
| C | 0.272 | 0.155 | 0.135 | 0.133 | 0.137 | 0.125 | 0.134 | 0.109 | 0.101 | 0.106 |
| D | 0.273 | 0.229 | 0.209 | 0.190 | 0.170 | 0.139 | 0.086 | 0.103 | 0.089 | 0.078 |
| E | 0.335 | 0.242 | 0.233 | 0.190 | 0.139 | 0.179 | 0.108 | 0.105 | 0.111 | 0.109 |
| F | 0.298 | 0.287 | 0.189 | 0.130 | 0.089 | 0.114 | 0.106 | 0.113 | 0.077 | 0.089 |
| G | 0.117 | 0.118 | 0.054 | 0.049 | 0.041 | 0.032 | 0.043 | 0.038 | 0.032 | 0.039 |

A: No treatment control
B: $C_8$ sodium acyl lactylate
C: sodium $C_{12}$ acyl lactylate
D: $C_{14}$ sodium acyl lactylate
E: sodium $C_{16}$ acyl lactylate
F: $C_{18}$ sodium acyl lactylate
G: sodium lactate

Example 2. Penetration and Hydrolysis of lactylate

Sodium lauroyl lactylate radiolabelled with $^{14}C$ was prepared from labelled lactic acid. A comparison was made between this material and the radiolabelled starting material.

The test material was dissolved in water at a concentration of 55.6mM corresponding to 1.63% by weight. The radiolabelled lactic acid was neutralised to its sodium salt, and diluted to 55.6mM. 30µl quantities of solution were applied to test pieces of skin from pig ear, as in the previous example. After incubation for 15 minutes the excess solution was removed with cotton wool.

Ten tape strips were taken, as in the previous example. Each tape strip was placed in a vial with 1ml of phosphate buffer and 10ml of scintillation fluid. The radiolabelled content was measured using a scintillation counter. The results are set out in the following Table:

| Tape No | Scintillation Count (thousands) | |
|---|---|---|
| | Lactate | Lauroyl lactylate |
| 1 | 36.1 | 37.1 |
| 2 | 19.1 | |
| 3 | 14.3 | 5.6 |
| 4 | 9.0 | 5.2 |
| 5 | 5.1 | 4.2 |
| 6 | 4.2 | 4.4 |
| 7 | 4.5 | 7.7 |
| 8 | 2.8 | 8.3 |
| 9 | 2.7 | 8.6 |
| 10 | 2.1 | 19.0 |

These results show that lauroyl lactylate penetrated more deeply into the skin than lactate itself.

In a similar experiment nine tape strips were taken after six hours. The first three strips were incubated in 3ml of phosphate buffer. The next three strips and the final three strips were likewise incubated. After 30 minutes the buffer solutions were spotted onto a thin-layer chromatography plate (bearing silica gel as the stationary phase).

The plate was eluted with 80:20:2 hexane:ether:acetic acid mixture. The spots on the plate were detected and quantified by their emitted radiation. Each lane on the plate displayed two spots, one corresponding to hydrolysis products of the lauroyl lactylate, and a second spot corresponding to the lauroyl lactylate itself. The results (mean of three repeats) were:

| | % of total label |
|---|---|
| Tapes 1-3 | |
| peak 1 | 13.1 |
| peak 2 | 8.9 |
| Tapes 4-6 | |
| peak 1 | 35.1 |
| peak 2 | 64.9 |
| Tapes 7-9 | |
| peak 1 | 45.2 |
| peak 2 | 54.8 |

These results show that hydrolysis of the lactoyl lactylate had occurred in each instance and had occurred to the greatest extent with the material which had penetrated furthest into the skin.

Example 3. Penetration and Hydrolysis of Glycolate

Sodium dodecyl glycolate, radiolabelled on the glycolate moiety, was prepared from radiolabelled glycolic acid. It was make up into a 55.6mM solution in demineralised water, which is a concentration of 1.56% by weight.

The penetration during 15 minutes and hydrolysis over six hours of this dodecyl glycolate was determined using pig skin as in the preceding example. The scintillation counts, showing the extent of penetration are set out in the following Tables.

| Tape No | Scintillation count (thousands) |
|---|---|
| 1 | 37.7 |
| 2 | 19.6 |
| 3 | 13.4 |
| 4 | 8.6 |
| 5 | 9.2 |
| 6 | 6.8 |
| 7 | 7.4 |
| 8 | 4.9 |
| 9 | 8.1 |
| 10 | 5.7 |

The results of thin layer chromatography, showing the extent of hydrolysis were:

| | % of total label |
|---|---|
| Tapes 1-3 | |
| peak 1 | 58.3 |
| peak 2 | 41.7 |
| Tapes 4-6 | |
| peak 1 | 51.3 |
| peak 2 | 48.7 |
| Tapes 7-9 | |
| peak 1 | 71.6 |
| peak 2 | 28.4 |

As with the lactylate in the previous example, these results show that hydrolysis takes place, especially at the level (Tapes 7 to 9) furthest into the skin.

Example 4. Penetration of Lactylate

Radiolabelled sodium lauroyl lactylate, as used in Example 2, was incorporated into a detergent composition containing

| | % w/w |
|---|---|
| Sodium laurylether sulphate | 10.5 |
| Cocoamidopropyl betaine | 8.5 |
| Sodium lauroyl lactylate | 18.0 |
| Glycerol | 5.0 |
| Ethoxylated fatty alcohol | 8.0 |
| Vitamin E acetate | 0.1 |
| Cationic polymer (modified guar gum) | 0.2 |
| Water | balance |

This solution was diluted with about four times its own volume of water, so that the concentration of sodium lauroyl lactylate was 0.12M.

100 µl quantities of this diluted solution were applied to test pieces of pig ear, left for 10 seconds and rinsed off. Excess water was removed with cotton wool, and nine tape strips were taken. All the tape strips were placed in a

vial with phosphate buffer and scintillation fluid. The radiolabel content was measured using a scintillation counter. This showed that a small proportion of the radiolabelled lactylate had deposited onto the skin, despite the presence of other surfactant and short contact time.

Example 5. Demonstrating Moisturisation of the Skin

The topical application to skin of hydroxyalkanoate derivatives improves the retention of moisture by the skin and/or increases the level of moisture in the skin to a significant extent. This has been demonstrated by employing a panel of human volunteers who receive prescribed amounts of test and control products, in accordance with a standardised procedure, after which conductivity measurements at the skin surface are made and recorded as an index of the benefit or otherwise of the applied product. An increase in skin conductivity is thus used as an index of increase in skin moisturisation.

The procedure for carrying out this evaluation and the measurement of skin conductivity will now be described.

Measurement of Skin Conductivity

This method measures the cumulative effect of applying products to the skin, on the conductivity of the superficial stratum corneum.

It is well established that the conductivity of the skin, as measured by placing the probe of a suitable conductivity meter in contact with the skin, is proportional to the water content of the outermost strata of the epidermis. The measurement of skin conductivity can therefore be used to indicate the ability of a topically applied product to hydrate the epidermis: see D. van Neste, J. Dermatol Sci., 2, p119 - 124, (1991), "Comparative Study of Normal and Rough Human Skin Hydration in vivo: evaluation with four different instruments". The method is widely used and accepted as indicative of product skin moisturisation efficacy.

The device for measuring skin conductivity employed in this test is the SKICON 200, a skin hygrometer available from IBS Co.

A small panel of volunteers, greater than six in number and preferably from 12 to 20, is recruited for the test.

Two sites 3 x 4cm are marked onto each volar forearm at least 6cm from either the wrist or elbow and with a separation of at least 6cm. Each site is treated once a day for five consecutive days with 100µl of a single treatment material. Allocation of material to left or right arm, upper or lower site is varied to avoid site effects. A total of two treatment materials can be used, one site per panellist remaining untreated for the entire five day period.

On the first day of the test, panellists are allowed to equilibrate in a climate controlled environment (20°C 70% RH for 15 minutes with no significant sensory stimuli). Eight conductivity measurements are taken per site using random positions within the test site; these represent the baseline. 100µl of treatment material is then applied and gently rubbed into the test site for each of the three treatment sites. Further four sets of eight readings from the treatment sites are then taken one, two and four hours after application, again using the equilibration period in a climate controlled environment. This procedure is repeated on the following four days.

The effect of each treatment is then assessed using the following method:

$$\text{adjusted effect} = \frac{\text{Mean treatment site reading}}{\text{Mean pretreatment site reading}}$$

This value is calculated for each treatment, for each timepoint on each day of treatment for each panellist, resulting in a final mean value for the adjusted effect for the entire panel also being obtained by calculation.

Skin Moisturisation using Sodium Dodecyl Lactylate

The following treatments were applied in this test

1. 5mM Sorensons phosphate buffer pH 7.00 + 2% Nipagin
2. 100mM Sodium dodecoyl lactylate in buffer (a concentration of 3.0%)
3. No treatment

The results obtained were as follows

The results are shown in the table below which records the mean adjusted SKICON 200 readings (i.e. proportional to initial skin conductivity = moisturisation) at hourly periods up to 4 hours.

| | T=0 Hr | T=1 Hr | T=2 Hr | T=4 Hr |
|---|---|---|---|---|
| Buffer | 1.00 | 1.09 | 1.10 | 1.10 |
| Na Acyl Lactylate | 1.00 | 1.27 | 1.30 | 1.31 |
| No Treatment | 1.00 | 1.05 | 1.04 | 1.03 |
| Sig. Diff. P=0.05 | | 10.19 | 0.20 | 0.19 |

The above results are also shown in Figure 2, which plots the Mean Adjusted SKICON 200 values on the abscissa, against time in hours on the ordinate.

Conclusions

It can be concluded from these results that significant moisturisation of the skin was achieved following topical application of the acyl lactylate as compared with the use of the buffer alone.

Example 6

An aftershave gel was prepared with the following formulation:

| Ingredients | % w/w |
|---|---|
| Ethanol | 40 |
| Glycerol | 2 |
| Ethoxylated fatty alcohols | 4 |
| iso stearoyl lactylate | 0.1 |
| Silicone oil | 3 |
| Carpobol 980 | 0.8 |
| Vitamin E acetate | 0.1 |
| Triethanolamine | 1.4 |
| Water | balance to 100% |

An aftershave lotion was prepared with the following formulation:

| Ingredients | % w/w |
|---|---|
| Ethanol | 40 |
| Glycerol | 2 |
| Ethoxylated fatty alcohols | 4.0 |
| iso-stearoyl lactylate | 0.5 |
| Carpobol 980 (polyacrylate thickener) | 0.8 |
| Triethanolamine | 0.15 |
| Water | balance to 100% |

The above gel and lotion were tested for skin moisturising and lactate delivery properties, using the volar forearms of a panel of human volunteers.

Each test site was divided into two regions (upper and lower). This allows both products to be applied to separate skin areas of each panellist and compared with no-treatment control areas.

Panellists were instructed not to use any other cleansing or care products on the test areas. A standard quantity of each product was applied by the panellist twice a day for five days with a five hour interval.

Skin conductivity was measured using the Skicon 200 instrument, as described in Example 5.

Measurement was carried out before the first application, then 1 hour after the second application on each day. The results, quoted relative to the initial conductivity measurement, are given in the table below and show that the products of the invention gave markedly enhanced conductivity relative to the no-treatment controls.

Lactate in the upper stratum corneum was determined by the tape stripping method generally as described in Example 1, at the end of the test. Five strips were taken, placed in a single tube and analysed for total lactate content.

The results obtained were:

| | Mean absorption at 540mm |
|---|---|
| Before treatment | 0.59 |
| No-treatment control | 0.55 |
| Aftershave gel | 0.67 |
| Aftershave lotion | 0.66 |

It can be seen that both products gave an increase in lactate content of the skin. The size of the increase was sufficiently large to be statistically significant.

The invention is further illustrated by reference to the following examples.

Example 7

Lauryl lactate, having the formula

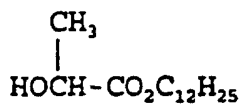

was dispersed in water at a concentration of 2% by weight.

This dispersion was tested on the volar forearm of a human volunteer. 100 µl of the dispersion was applied to each of three areas ($15cm^2$) on the volar forearm, rubbed in and allowed to dry. Ten Desquarne tape strips were taken fropm a fourth, untreated area. After 1 hour ten tape strips were taken from one treated area. Ten tape strips were taken from the other treated areas after 3 and 8 hours respectively. The tape strips taken after 1 hour and 3 hours were stored at room temperature for the rest of the 8 hour period, allowing enzymic hydrolysis.

The amount of lactic acid on each tape strip was determined, by the procedure quoted in Example 1. The results are set out in the following table and show that the application of the ester increased lactate levels in the skin. Moreover, this is at all levels, indicating penetration of the lactate ester into the skin where it is hydrolysed.

| Tape No | No Treatment | 1 Hour | 3 Hours | 8 Hours |
|---|---|---|---|---|
| 1 | 0.172 | 0.289 | 0.282 | 0.260 |
| 2 | 0.209 | 0.361 | 0.250 | 0.210 |
| 3 | 0.197 | 0.217 | 0.252 | 0.250 |
| 4 | 0.199 | 0.124 | 0.262 | 0.222 |
| 5 | 0.189 | 0.192 | 0.485 | 0.259 |
| 6 | 0.177 | 0.201 | 0.487 | 0.230 |
| 7 | 0.183 | 0.179 | 0.271 | 0.241 |
| 8 | 0.177 | 0.177 | 0.246 | 0.234 |
| 9 | 0.157 | 0.211 | 0.254 | 0.194 |
| 10 | 0.177 | 0.169 | 0.269 | 0.449 |

Example 8. Water-in-oil skin cream

| Ingredients | % w/w |
|---|---|
| Silicone oil | 20.00 |
| Sodium chloride | 2.00 |

(continued)

| Ingredients | % w/w |
|---|---|
| Sodium octanoyl lactylate | 1.00 |
| Whitener | 0.15 |
| Preservatives | 0.36 |
| Sodium hydroxide | 1.00 |
| Water | to 100.00 |

Example 9. Water-in-oil skin cream

| Ingredients | % w/w |
|---|---|
| Silicones | 20.50 |
| Whitener | 0.20 |
| Preservatives | 0.30 |
| Perfume | 0.15 |
| Ammonium hydroxide | 7.95 |
| Decanoyl lactylic acid | 1.00 |
| Humectant | 10.00 |
| Ammonium chloride | 2.00 |
| Water | to 100.00 |

Example 10. Water-in-oil skin cream with sunscreens

| Ingredients | % w/w |
|---|---|
| Silicones | 24.00 |
| whitener | 0.10 |
| Preservatives | 0.01 |
| Potassium lauroyl lactylate | 1.50 |
| Potassium chloride | 1.50 |
| Humectants | 5.00 |
| Evening primrose oil | 3.00 |
| Sunscreens | 4.00 |
| Bactericides | 0.30 |
| Water | to 100.00 |

Example 11. Oil-in-water skin cream

| Ingredients | % w w |
|---|---|
| Emulsifier | 10.00 |
| Silicone oil | 8.00 |
| Thickener | 0.50 |
| Whitener | 0.10 |
| Preservatives | 0.10 |
| Octanoyl lactylic acid | 2.00 |
| Humectant | 10.00 |

(continued)

| Ingredients | % w w |
|---|---|
| Evening primrose oil | 2.00 |
| Sunscreens | 3.00 |
| Bactericides | 0.30 |
| Triethanolamine | 3.10 |
| Water | to 100.00 |

Example 12. Face mask

| Ingredients | % w/w |
|---|---|
| Kaolin | 35.00 |
| Bentonite | 5.00 |
| Cetyl alcohol | 2.00 |
| Potassium myristoyl lactylate | 5.25 |
| Glycerol | 10.00 |
| Methyl paraben | 0.10 |
| Potassium dodecyl sulphate | 2.00 |
| Perfume | 0.75 |
| Water | to 100.00 |

Example 13. Cleansing Cream

| Ingredients | % w/w |
|---|---|
| Lanette wax SX | 16.00 |
| Mineral oil | 20.00 |
| Microcrystalline wax | 3.00 |
| Glycerol | 5.00 |
| Sodium isostearoyl lactylate | 1.00 |
| Water | 55.00 |
| Perfume | |
| Preservative | |

Example 14. Cleansing Milk

| Ingredients | % w/w |
|---|---|
| Mineral oil | 10.00 |
| Cetyl alcohol | 0.50 |
| Stearic acid | 3.00 |
| Sodium isostearoyl lactylate | 1.00 |
| TEA | 1.80 |
| Water | 83.7 |
| Perfume | q.s |
| Preservative | q.s |

Example 15. Cleansing Mousse

| Ingredients | % w/w |
|---|---|
| Sodium lauryl ether sulphate (28%) | 18.00 |
| Sodium cocoamidopropyl betaine | 7.50 |
| Sodium lauroyl lactylate | 1.00 |
| Glycerol | 10.00 |
| Ethanol | 5.00 |
| Vitamin E acetate | 0.10 |
| Cremophore RH410 | 0.50 |
| Redoderm LIS 80 | 1.00 |
| Preservative | 0.26 |
| Ammonium hydroxide (29%) to pH 7.00 | |
| Colourant | q.s |
| Perfume | q.s |
| Propane/Butane | 3.00 |
| Water | to 100.00 |

Example 16. Mild Facial Cleanser

| Ingredients | % w/w |
|---|---|
| Sodium lauroyl lactylate | 20.00 |
| Glycerol | 10.00 |
| Sodium cocoyl isethionate | 7.00 |
| Cocamidopropyl betaine | 4.00 |
| Polyoxeyethylene (EO) 20 sorbitan monolaurate | 3.00 |
| Hydroxypropyl methylcellulose | 0.20 |
| Preservative | 0.20 |
| Perfume | 0.10 |
| Citric acid to pH 6.50 | |
| Water | to 100.00 |

Example 17. Liquid Soap

| Ingredients | % w/w |
|---|---|
| Stearoyl lactylate | 15.00 |
| Triethanolammonium N-lauroyl glutamate | 9.00 |
| Cocoamidopropyl betaine | 4.00 |
| Propyleneglycol hydroxy isostearate | 1.00 |
| Trisodium citrate | 7.00 |
| Preservative | 0.26 |
| Perfume | 0.15 |
| Triethanolamine to pH 7.00 | |
| Water | to 100.00 |

Example 18. Cleansing Beauty Bar

| Ingredients | % w/w |
|---|---|
| Sodium n-myristoyl dilactylate | 15.00 |
| Sodium n-palmitoyl dilactylate | 15.00 |
| Sodium n-stearoyl aspartate | 10.00 |
| Glycerol | 8.00 |
| Diglycerol | 1.00 |
| Perfume | 0.50 |
| Preservatives | 0.26 |
| Colourant | 0.10 |
| Water | to 100.00 |

Example 19. Deep Cleansing Gel

| Ingredients | % w/w |
|---|---|
| Mineral oil | 35.00 |
| Lanolin | 10.00 |
| Coco diethanolamide | 4.00 |
| Stearic acid | 2.43 |
| Oleic acid | 3.64 |
| Sodium stearoyl lactylate | 1.00 |
| Sodium hydroxide | 0.38 |
| Perfume, preservative and colourant | q.s. |
| Water | 43.29 |

Example 20. Moisturising Lotion

| Ingredients | % w/w |
|---|---|
| Glycerol | 5.00 |
| TEA | 1.00 |
| Sodium isostearoyl lactylate | 1.00 |
| Magnesium aluminium silicate | 4.00 |
| Glyceryl monostearate | 0.70 |
| Mineral oil | 3.00 |
| Stearic acid | 2.50 |
| Cetyl alcohol | 0.30 |
| Cerasynt IP | 1.50 |
| Silicone oil | 1.00 |
| Preservative | 0.26 |
| Perfume | 0.25 |
| Carbopol 934 (2%) | 3.00 |
| Water | to 100.00 |

Example 21. Skin Cream

| Ingredients | % w/w |
|---|---|
| Lanette wax SX | 10.00 |
| Cetyl alcohol | 4.00 |
| Mineral oil | 4.00 |
| Glycerol | 6.00 |
| Calcium stearoyl lactylate | 1.00 |
| Preservative | 0.26 |
| Perfume | 0.30 |
| Water | to 100.00 |

Example 22 - Dry Skin Cream

| Ingredients | % w/w |
|---|---|
| Mineral oil | 8.00 |
| Petrolatum | 4.50 |
| Ceresin wax | 1.75 |
| Candilla wax | 1.00 |
| Glyceryl monostearate | 1.75 |
| Stearic acid | 2.25 |
| Cetyl alcohol | 1.40 |
| Isopropyl palmitate | 6.00 |
| Laureth - 23 | 0.36 |
| Sorbitan oleate | 1.40 |
| Preservative | 0.26 |
| Glycerol | 3.00 |
| Sodium isostearoyl lactylate | 1.00 |
| Sodium stearoyl lactylate | 0.50 |
| Sodium palmitoyl lactylate | 0.50 |
| TEA | 0.60 |
| Carbopol 934 (2%) | 10.00 |
| Trisodium EDTA | 0.03 |
| Perfume | 0.30 |
| Water | to 100.00 |

Example 23. Barrier Cream

| Ingredients | % w/w |
|---|---|
| Stearic Acid | 6.00 |
| Cetyl alcohol | 3.00 |
| Lanolin | 3.00 |
| Soft paraffin | 2.00 |
| Calcium stearoyl lactylate | 0.50 |
| Sodium hydroxide | 0.65 |
| Kaolin | 18.00 |

(continued)

| Ingredients | % w/w |
|---|---|
| Perfume | 0.30 |
| Colourant | 0.10 |
| Preservative | 0.20 |
| Water | to 100.00 |

Example 24. Anti-ageing Eye Gel

| Ingredients | % w/w |
|---|---|
| Glycerol | 3.00 |
| Sodium lauroyl lactylate | 0.50 |
| Vitamin E acetate | 0.10 |
| Aloe vera gel | 0.25 |
| TEA to pH 7.00 | |
| Carbopol 934 (2%) | 25.00 |
| Preservative | 0.15 |
| Perfume | q.s. |
| Colourant | q.s. |
| Water | to 100.00 |

Example 25. Aftershave Lotion

| Ingredients | % w/w |
|---|---|
| Ethanol | 40 |
| Glycerin | 2 |
| Iso-stearoyl lactylate | 0.5 |
| PEG hydrogenated castor oil | 5 |
| Perfume | 3 |
| Water | to 100 |

Example 26. Aftershave Gel

| Ingredients | % w/w |
|---|---|
| Ethanol | 30 |
| Glycerin | 2 |
| Iso-stearoyl lactylate | 0.1 |
| PEG hydrogenated castor oil | 0.55 |
| Vitamin E acetate | 0.1 |
| Emollient oil | 1 |
| Carbopol 980 | 1 |
| Triethanolamine | 2 |
| Perfume | 0.4 |
| Perfume solubiliser | 0.5 |
| Water | to 100 |

Example 27. Aftershave Lotion

| Ingredients | % w/w |
|---|---|
| Ethanol | 40 |
| Glycerin | 2 |
| Iso-stearoyl lactylate | 1 |
| PEG hydrogenated castor oil | 0.55 |
| Vitamin E acetate | 0.1 |
| Carbopol 980 | 0.6 |
| Triethanolamine | 1.2 |
| Perfume | 3 |
| Perfume solubiliser | 5 |
| Water | to 100 |

Example 28. Skin Cleansing Milk

| Ingredients | % w/w |
|---|---|
| PEG-20 Sorbitan Monostearate | 1.80 |
| Sorbitan monostearate | 1.50 |
| Mineral Oil | 39.9 |
| Lauryl lactate | 5.00 |
| Triethanolamine | 0.70 |
| Stearic Acid | 1.40 |
| Porpylparaben | 0.10 |
| Polyethylene glycol | 8.00 |
| Methyl paraben | 0.10 |
| Ethylhydroxyethyl cellulose | 0.54 |
| Sorbic acid | 0.002 |
| Water | to 100 |

Example 29. Dry Skin Cream

| Ingredients | % w/w |
|---|---|
| Mineral Oil | 6.00 |
| Isostearyl lactate | 2.00 |
| Cerasin Wax | 1.75 |
| Petroleum jelly | 4.50 |
| Glyceryl monostearate | 1.75 |
| Candelilla Wax | 1.00 |
| Stearic acid | 2.25 |
| Cetyl alcohol | 1.40 |
| Isopropyl palmitate | 6.00 |
| Laureth 23 | 0.36 |
| Sorbitan oleate | 1.40 |
| Propyl paraben | 0.10 |
| Glycerol | 3.00 |

(continued)

| Ingredients | % w/w |
|---|---|
| Triethanolamine | 0.60 |
| Methyl paraben | 0.15 |
| Carbopol CC-122 (2% dispersion) | 10.0 |
| Imidazolidinyl urea | 0.20 |
| Water | to 100 |

This composition can be modified by increasing the content of isostearyl lactate to 3% or 4% of the composition, and reducing the mineral oil to 5% or 4%.

Example 30. Anti-aging Sunscreen

| Ingredients | % w/w |
|---|---|
| Propyl paraben | 0.10 |
| Dea-cetyl phosphate | 1.00 |
| Benzophenone-3/oxobenzone | 6.00. |
| PVP/eicosene copolymer | 2.50 |
| Stearic acid | 4.00 |
| Tocopherol acetate | 0.10 |
| Dimethicone | 1.00 |
| Stearyl lactate | 1.50 |
| Petroleum Jelly | 0.25 |
| Octyl methoxycinnamate ethylhexyl P-ME | 7.50 |
| Triethanolamine | 1.40 |
| DMDM hydantoin | 0.25 |
| Carbopol 2% dispersion | 5.00 |
| Magnesium aluminium silicate | 0.20 |
| Glycerol | 5.00 |
| Methyl paraben | 0.15 |
| Aloe Vera Gel | 0.05 |
| Disodium EDTA | 0.05 |
| Simethicone | 0.01 |
| Cetyl alcohol | 0.70 |
| Octyl salicylate/2 ethylhexyl salicylate | 5.00 |
| Water | to 100 |

Example 31. Moisturising Facial Cleanser

| Ingredients | % w/w |
|---|---|
| Mineral Oil | 7.00 |
| Lauryl lactate | 2.00 |
| Sorbitan stearate | 1.50 |
| Polysorbate 60 | 1.80 |
| Methyl paraben | 0.20 |
| Propyl paraben | 0.10 |
| PEG 550 | 8.00 |

(continued)

| Ingredients | % w/w |
|---|---|
| Triethanolamine | 1.06 |
| Stearic acid | 1.40 |
| BHT | 0.05 |
| Phenoxyethanol | 0.40 |
| Carbopol 2% dispersion | 5.00 |
| Glycerol | 5.00 |
| Water | to 100 |

This cleanser can be modified by reducing the mineral oil to 5% and increasing the lauryl lactate to 4%.

Example 32. Smoothing Shaving Foam

| Ingredients | % w/w |
|---|---|
| Stearic acid | 3.27 |
| Palmitic acid | 3.51 |
| Lauric acid | 0.76 |
| Triethanolamine | 3.01 |
| Potassium hydroxide | 0.28 |
| Glycerol | 4.61 |
| Lauryl lactate | 1.30 |
| Tween 20 | 0.96 |
| Tocopherol acetate | 0.05 |
| Isostearyl lactylate | 0.10 |
| Silicone Fluid DC2-1865 | 1.50 |
| Silicone Fluid DC193 | 0.77 |
| CAP 48 | 4.00 |
| Water | to 100 |

Example 33. Aftershave Balm

| Ingredients | % w/w |
|---|---|
| Ethanol | 45.0 |
| Cetyl lactate | 0.40 |
| Glycerol | 2.00 |
| Perfume | 0.40 |
| Benzophenone-11 | 0.02 |
| Water | to 100 |

Example 34. Rinsable Cream Cleanser

| Ingredients | % w/w |
|---|---|
| Mineral Oil | 30.00 |
| Myristyl lactate | 10.00 |

(continued)

| Ingredients | % w/w |
|---|---|
| Beeswax | 2.30 |
| Cerasin | 0.50 |
| Propyl paraben | 0.10 |
| PEG-16 soya sterol | 5.00 |
| PEG-8 dilaurate | 2.00 |
| Cetearyl alcohol | 0.80 |
| Behenic acid | 0.80 |
| Methyl paraben | 0.15 |
| Disodium EDTA | 0.05 |
| Sodium borate | 0.80 |
| Carpobol 2% dispersion | 16.00 |
| Water | to 100 |

## Claims

**1.** A composition suitable for topical application to the skin for delivering to the epidermis a 2-hydroxy alkanoate having the structure (2):

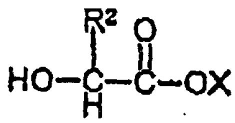

(2)

where

$R^2$ represents H- or $CH_3$,
X represents H- or a counterion,

thereby moisturising the skin, the composition comprising:

i) an effective amount of a hydroxyalkanoate derivative possessing the ability of penetrating the stratum corneum and hydrolysing by enzymatic cleavage within the epidermis to provide the 2-hydroxyalkanoate of structure (2), wherein the hydroxyalkanoate derivative has the structure (1):

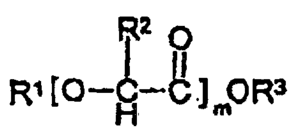

(1)

where

$R^1$ represents H- or $C_xH_yO_zN_w$-C(O)-,
$R^2$ represents H- or $-CH_3$,
$R^3$ represents H-, $C_xH_yO_zN_w$- or a metallic, ammonium or alkanolantmonium counterion,
x is an integer of from 1 to 20,
y is an integer of from 3 to 41,
z is 0, or an integer of from 1 to 10,
w is 0, or an integer of from 1 to 5, and
m is an integer of from 1 to 5,

provided that when $R^1$ is H-, then $R^3$ is $C_xH_yO_xN_w$-, or when $R^3$ is H- or a counterion, then $R^1$ is $C_xH_yO_zN_w$-C (O) -;

ii) a cosmetically acceptable vehicle for the hydroxy alkanoate derivative; and

iii) a water immiscible oil in an amount exceeding the amount of the hydroxyalkanoate derivative having the structure(1); and

iv) a penetration enhancer selected from the group consisting of

2-methyl propan-2-ol
Propan-2-ol
Hexan-2,5-diol
POE(2) ethyl ether
Di(2-hydroxypropyl) ether
Pentan-2,4-diol
POE(2) methyl ether
Propan-1-ol
1,4 Dioxane
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Dibenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl myristate
isopropyl palmitate
Ethyl laurate
2-ethyl-hexyl pelargonate
Isopropyl isostearate
Butyl laurate
Benzyl benzoate
Butyl benzoate
Hexyl laurate
Ethyl caprate
Ethyl caprylate
Butyl stearate

Benzyl salicylate
2-Pyrrolidone
1-Methyl-2-pyrrolidone
5-Methyl-2-pyrrolidone
1,5-Dimethyl-2-pyrrolidone
1-Ethyl-2-pyrrolidone
Tetrahydrofurfural alcohol
Urea
Diethyl-m-toluamide; and
1-Dodecylazacyloheptan-2-one

**2.** A composition as claimed in Claim 1 wherein the water immiscible oil comprises mineral oil, vegetable oils or silicone oils.

**3.** A composition as claimed in Claim 1 or Claim 2 wherein the hydroxyalkanoate derivative is an acyl hydroxyalkanoate, where $R^1$ in structure (1) is represented by the saturated or unsaturated acyl group:

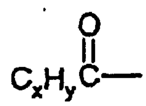

and where

$R^2$ represents -H or $-CH_3$,
m is 1 to 5, and
$R^3$ represents -H or an alkali metal, an alkaline earth metal, ammonium or alkanol ammonium counterion.

**4.** A composition as claimed in Claim 3, where $R^1$ is chosen from:

n-propionyl
n-butanoyl
n-hexanoyl
n-decanoyl
n-dodecanoyl
n-tetradecanoyl
n-hexadecanoyl and
n-octadecanoyl

**5.** A composition as claimed in Claim 3 wherein $R^1$ in structure (1) represents the branched chain acyl group:

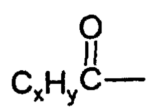

where

$C_xH_y$- represents a branched chain alkyl group and m is 1.

**6.** A composition as claimed in Claim 5, where $R^1$ is chosen from:

iso-butanoyl
iso-hexanoyl
iso-octanoyl and
iso-octadecanoyl.

**7.** A composition as claimed in Claims 1 or 2, wherein the hydroxyalkanoate derivative is an acyl hydroxyalkanoate

where $R^1$ in structure (1) represents the saturated or unsaturated acyl group

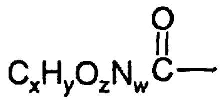

where

$R^2$ represents -H or $-CH_3$,
m is from 1 to 5,
$R^3$ represents -H, or an alkali metal, an alkaline earth metal, ammonium or alkanol ammonium countërion,
z is 0, or an integer of from 1 to 10,
w is 0, or an integer of from 1 to 5, provided that z and w do not both represent 0 simultaneously.

**8.** A composition as claimed in Claim 7, where $R^1$ is chosen from:

ethylglycoloyl
leucoyl
mandeloyl
cocoamidopropanoyl
pyroglutamoyl
cholesteroyl and
ceramidoyl

**9.** A composition as claimed in Claim 1 or 2 wherein the hydroxyalkanoate derivative is an alkyl hydroxyalkanoate where

$R^1$ represents H-
$R^2$ represents H- or $-CH_3$
$R^3$ represents $C_xH_y$-, and
m is 1.

**10.** A composition as claimed in Claim 9 in which the alkyl hydroxyalkanoate is chosen

methyl glycate
n-butyl glycate
n-hexyl glycate
n-octyl glycate
n-decyl glycate
n-dodecyl glycate
n-octadecyl glycate
methyl lactate
n-butyl lactate
n-hexyl lactate
n-octyl lactate
n-decyl lactate
n-dodecyl lactate
n-tetradecyl lactate
n-hexadecyl lactate
n-octadecyl-lactate, and
octyldecyl lactate.

**11.** A composition as claimed in any one of the preceding claims wherein the value of x is at least 6.

**12.** A composition as claimed in any one of the preceding claims wherein the amount of hydroxyalkanoate derivative present in the composition as an effective amount, is from 0.001 to 50%, preferably. from 0.1 to 30%, and most

preferably from 0.5 to 20% by weight of the composition.

**13.** A composition as claimed in Claim 12 wherein the amount of hydroxyalkanoate derivative present in the composition is from 0.05 to 2% by weight of the composition.

**14.** A composition as claimed in any one of the preceding claims wherein the cosmetically acceptable vehicle includes water in an amount which is at least 43% of the composition and/or includes aqueous ethanol in an amount which is at least 50% by weight of the composition, the amount of ethanol, if any, not exceeding 50% by weight of the composition.

**15.** A composition as claimed in any one of the preceding claims wherein the composition comprises a retinoid chosen from

retinyl acetate
retinyl butyrate
retinyl propionate
retinyl octanoate
retinyl laurate
retinyl palmitate
retinyl oleate
retinyl linoleate, and
retinyl linolenate;

and/or comprises tocopherol and/or a tocopherol ester.

**16.** A composition as claimed in any one of the preceding claims wherein the composition is a leave-on product chosen from creams, lotions, milks, gels.

**17.** A composition as claimed in any one of the preceding claims wherein the composition comprises an ionic surfactant other than a said hydroxyalkanoate derivative.

**18.** A composition as claimed in any one of the preceding claims wherein the composition is a pre-shave or after-shave product.

**19.** A composition as claimed in any one of the preceding claims wherein the composition is applied to the human face.

**20.** Use of a hydroxy alkanoate derivative having structure (1) as defined in anyone of the preceding claims in the manufacture of a composition for delivering to the epidermis a 2-hydroxy alkanoate having the structure (2):

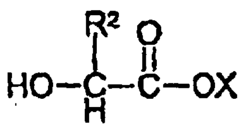 (2)

where

$R^2$ represents H- or $CH_3$,
X represents H- or a counterion,

which comprises the steps of

(i) applying topically to the skin the composition including the hydroxyalkanoate derivative having structure (1):
(ii) leaving the composition in contact with the skin for at least 10 seconds to permit the hydroxyalkanoate derivative to penetrate through the stratum corneum to reach the lower strata of the epidermis, and
(iii) cleaving the hydroxyalkanoate derivative in the epidermis by contact with epidermal esterases to provide the 2-hydroxy alkanoate having the structure (2).

## Patentansprüche

**1.** Zusammensetzung, die für die topische Austragung auf die Haut geeignet ist, für die Abgabe eines 2-Hydroxyalkanoats mit der Struktur (2) an die Epidermis:

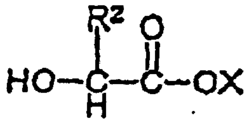 (2)

worin

$R^2$ H- oder $CH_3$ darstellt,
X H- oder ein Gegenion darstellt,

wodurch die Haut befeuchtet wird, wobei die Zusammensetzung umfaßt:

i) eine wirksame Menge eines Hydroxyalkanoat-Derivats, das die Fähigkeit besitzt, die Hornschicht der Haut zu durchdringen und durch enzymatische Spaltung die Epidermis zu hydrolysieren, wodurch das 2-Hydroxyalkanoat mit der Struktur (2) bereitgestellt wird, worin das Hydroxyalkanoat-Derivat die Struktur (1) aufweist:

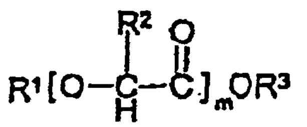 (1)

worin

$R^1$ H- oder $C_xH_yO_zN_w$-C(O)- darstellt,
$R^2$ H- oder -$CH_3$ darstellt,
$R^3$ H-, $C_xH_yO_zN_w$- oder ein metallisches, Ammonium- oder Alkanolammonium-Gegenion darstellt,
x eine ganze Zahl von 1 bis 20 ist,
y eine ganze Zahl von 3 bis 41 ist,
z 0 oder eine ganze Zahl von 1 bis 10 ist,
w 0 oder eine ganze Zahl von 1 bis 5 ist und
m eine ganze Zahl von 1 bis 5 ist,

vorausgesetzt, daß, wenn $R^1$ H- ist, $R^3$ dann $C_xH_yO_zN_w$- ist oder, wenn $R^3$ H- oder ein Gegenion ist, $R^1$ dann $C_xH_yO_zN_w$-C(O)- ist;

ii) eine kosmetisch akzeptable Trägersubstanz für das Hydroxyalkanoat-Derivat; und

iii) ein mit Wasser mischbares Öl in einer Menge, die die Menge an dem Hydroxyalkanoat-Derivat mit der Struktur ( 1 ) übersteigt; und

iv) einen Durchdringungsverstärker, ausgewählt aus der Gruppe, bestehend aus 2-Methylpropan-2-ol, Propan-2-ol, Hexan-2,5-diol, POE(2)-Ethylether, Di(2-hydroxypropyl)ether, Pentan-2,4-diol, POE(2)-Methylether, Propan-1-ol, 1,4-Dioxan, Propylenglycoldipelargonat, Polyoxypropylen-15-Stearylether, Octylalkohol, POE-Ester von Oleylalkohol, Oleylalkohol, Laurylalkohol, Dioctyladipat, Dicapryladipat, Diisopropyladipat, Diisopropylsebacat, Dibutylsebacat, Diethylsebacat, Dimethylsebacat, Dioctylsebacat, Dibutylsuberat, Dioctylazelat, Dibenzylsebacat, Dibutylphthalat, Dibutylazelat, Ethylmyristat, Dimethylazelat, Butylmyristat, Dibutylsuccinat, Didecylphthalat, Decyloleat, Ethylcaproat, Ethylsalicylat, Isopropylmyristat, Isopropylpalmitat, Ethyllaurat, 2-Ethyl-hexylpelargonat, Isopropylisostearat, Butyllaurat, Benzylbenzoat, Butylbenzoat, Hexyllaurat, Ethylcaprat, Ethylcaprylat, Butylstearat, Benzylsalicylat, 2-Pyrrolidon, 1-Methyl-2-pyrrolidon, 5-Methyl-2-pyrrolidon, 1-Ethyl-2-pyrrolidon, 1-Ethyl-2-pyrrolidon, Tetrahydrofurfurylalkohol, Harnstoff, Diethyl-m-toluamid und 1-Dodecylazacyloheptan-2-on.

2. Zusammensetzung nach Anspruch 1, wobei das mit Wasser mischbare Öl Mineralöl, pflanzliche Öle oder Silikonöle umfaßt.

3. Zusammensetzung nach Anspruch 1 oder Anspruch 2, wobei das Hydroxyalkanoat-Derivat ein Acylhydroxyalkanoat ist, worin $R^1$ in Struktur (1) durch die gesättigte oder ungesättigte Acylgruppe dargestellt wird:

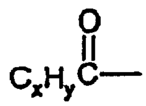

und worin

$R^2$ -H oder $-CH_3$ darstellt,
m 1 bis 5 ist und
$R^3$ -H oder ein Alkalimetall-, ein Erdalkalimetall-, Ammonium- oder Alkanolammonium-Gegenion ist.

4. Zusammensetzung nach Anspruch 3, worin $R^1$ ausgewählt ist aus:

n-Propionyl, n-Butanoyl, n-Hexanoyl, n-Decanoyl, n-Dodecanoyl, n-Tetradecanoyl, n-Hexadecanoyl und n-Octadecanoyl.

5. Zusammensetzung nach Anspruch 3, wobei $R^1$ in Struktur (1) die verzweigtkettige Acylgruppe darstellt:

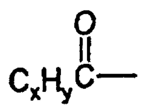

worin $C_xH_y$ eine verzweigtkettige Alkylgruppe darstellt und m 1 ist.

6. Zusammensetzung nach Anspruch 5, worin $R^1$ ausgewählt ist aus:

iso-Butanoyl, iso-Hexanoyl, iso-Octanoyl und iso-Octadecanoyl.

7. Zusammensetzung nach einem der Ansprüche 1 oder 2, wobei das Hydroxyalkanoat-Derivat ein Acylhydroxyalkanoat ist, worin $R^1$ in Struktur ( 1 ) die gesättigte oder ungesättigte Acylgruppe darstellt:

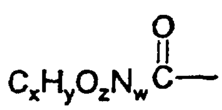

worin

$R^2$ -H oder $-CH_3$ darstellt,
m 1 bis 5 ist,
$R^3$ -H oder ein Alkalimetall-, ein Erdalkalimetall-, ein Ammonium- oder ein Alkanolammonium-Gegenion darstellt,
z 0 oder eine ganze Zahl von 1 bis 10 ist
w 0 oder eine ganze Zahl von 1 bis 5 ist,

vorausgesetzt, daß z und w nicht gleichzeitig 0 darstellen.

8. Zusammensetzung nach Anspruch 7, worin $R^1$ ausgewählt ist aus:

Ethylglycoloyl, Leucoyl, Mandeloyl, Cocoamidopropanoyl, Pyroglutamoyl, Cholesteroyl und Ceramidoyl.

**9.** Zusammensetzung nach Anspruch 1 oder 2, wobei das Hydroxyalkanoat-Derivat ein Alkylhydroxyalkanoat ist, worin
$R^1$ H- darstellt,
$R^2$ H- oder $-CH_3$ darstellt,
$R^3$ $C_xH_y$- darstellt und
m 1 ist.

**10.** Zusammensetzung nach Anspruch 9, worin das Alkylhydroxyalkanoat ausgewählt ist aus:

Methylglycat, n-Butylglycat, n-Hexylglycat, n-Octylglycat, n-Decylglycat, n-Dodecylglycat, n-Octadecylglycat, Methyllactat, n-Butyllactat, n-Hexyllactat, n-Octyllactat, n-Decyllactat, n-Dodecyllactat, n-Tetradecyllactat, n-Hexadecyllactat, n-Octadecyllactat und Octyldecyllactat.

**11.** Zusammensetzung nach einem der vorherigen Ansprüche, wobei der Wert von x zumindest 6 beträgt.

**12.** Zusammensetzung nach einem der vorherigen Ansprüche, wobei die Menge an dem in der Zusammensetzung als wirksame Menge vorliegenden Hydroxyalkanoat-Derivats 0,001 bis 50 Gew.-%, bevorzugt 0,1 bis 30 Gew.-% und am stärksten bevorzugt 0,5 bis 20 Gew.-% der Zusammensetzung beträgt.

**13.** Zusammensetzung nach Anspruch 12, wobei die Menge an dem in der Zusammensetzung vorliegenden Hydroxyalkanoat-Derivat 0,05 bis 2 Gew.-% der Zusammensetzung beträgt.

**14.** Zusammensetzung nach einem der vorherigen Ansprüche, wobei die kosmetisch akzeptable Trägersubstanz Wasser in einer Menge, die zumindest 43 % der Zusammensetzung ausmacht, umfaßt und/oder wässeriges Ethanol in einer Menge, die zumindest 50 Gew.-% der Zusammensetzung ausmacht, umfaßt, wobei die Menge an Ethanol, wenn vorhanden, 50 Gew.-% der Zusammensetzung nicht übersteigt.

**15.** Zusammensetzung nach einem der vorherigen Ansprüche, wobei die Zusammensetzung ein Retinoid, ausgewählt aus Retinylacetat, Retinylbutyrat, Retinylpropionat, Retinyloctanoat, Retinyllaurat, Retinylpalmitat, Retinyloleat, Retinyllinoleat und Retinyllinolenat umfaßt,
und/oder Tocopherol und/oder
einen Tocopherolester umfaßt.

**16.** Zusammensetzung nach einem der vorherigen Ansprüche, wobei die Zusammensetzung ein hinterbleibendes Produkt ist, ausgewählt aus Cremes, Lotionen, Milch, Gelen.

**17.** Zusammensetzung nach einem der vorherigen Ansprüche, wobei die Zusammensetzung ein ionisches oberflächenaktives Mittel umfaßt, daß ein anderes als das Hydroxyalkanoat-Derivat ist.

**18.** Zusammensetzung nach einem der vorherigen Ansprüche, wobei die Zusammensetzung ein Pre-Shave- oder ein After-Shave-Produkt ist.

**19.** Zusammensetzung nach einem der vorherigen Ansprüche, wobei die Zusammensetzung auf das menschliche Gesicht aufgetragen wird.

**20.** Verwendung eines Hydroxyalkanoat-Derivats mit der Struktur (1) wie in einem der vorherigen Ansprüche definiert, bei der Herstellung einer Zusammensetzung zur Abgabe eines 2-Hydroxyalanoats mit der Struktur (2) an die Epidermis:

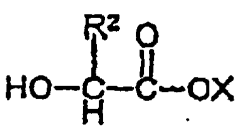 (2)

worin

$R^2$ H- oder $CH_3$ darstellt,
X H- oder ein Gegenion darstellt,

die die Schritte

(i) topische Auftragung der Zusammensetzung, die das Hydroxyalkanoat-Derivat mit der Struktur ( 1 ) umfaßt, auf die Haut;

(ii) Inkontakthalten der Zusammensetzung mit der Haut für mindestens 10 Sekunden, um zu ermöglichen, daß das Hydroxyalkanoat-Derivat durch die Hornschicht der Haut dringen kann, um den unteren Bereich der Epidermis zu erreichen und

(iii) Spalten das Hydroxyalkanoat-Derivats in der Epidermis durch Kontakt mit epidermalen Esterasen, um das 2-Hydroxyalkanoat mit der Struktur (2) bereitzustellen,

umfaßt.

## Revendications

**1.** Composition appropriée pour une application topique sur la peau pour délivrer sur l'épiderme un 2 - hydroxy alcanoate ayant la structure (2) :

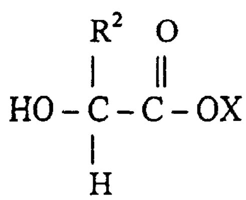

(2)

dans laquelle :

$R^2$ représente H - ou $CH_3$ ;
X représente H - ou un ion antagoniste,

pour hydrater ainsi la peau, la composition comprenant :

i) une quantité efficace d'un dérivé d'hydroxyalcanoate ayant la capacité de pénétrer la couche cornée et s'hydrolysant par clivage enzymatique dans l'épiderme afin de fournir le 2 - hydroxyalcanoate de structure (2), dans lequel le dérivé d'hydroxyalcanoate a la structure (1) :

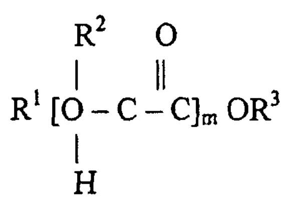

(1)

dans laquelle :

$R^1$ représente H - ou $C_xH_yO_zN_w$ - C (O) -,
$R^2$ représente H - ou - $CH_3$,
$R^3$ représente H -, $C_xH_yO_xN_w$ - ou un ion antagoniste métallique, ammonium ou alcanolammonium,

x est un nombre entier allant de 1 à 20,
y est un nombre entier allant de 3 à 41,
z est 0 ou un nombre entier allant de 1 à 10,
w est 0, ou un nombre entier allant de 1 à 5, et
m est un nombre entier allant de 1 à 5,

à condition que lorsque $R^1$ est H - alors $R^3$ est $C_xH_yO_zN_w$ - ou lorsque $R^3$ est H - ou un ion antagoniste, alors $R^1$ est $C_xH_yO_zN_w$- C (O) - ;

ii) un véhicule acceptable d'un point de vue cosmétique pour le dérivé hydroxy alcanoate ; et
iii) une huile non miscible dans l'eau, dans une quantité dépassant la quantité du dérivé hydroxy alcanoate ayant la structure (1) ; et
iv) un amplificateur de pénétration sélectionné à partir du groupe constitué du :

2 - méthyle - propane - 2 - ol ;
propane - 2 - ol ;
hexane - 2,5 - diol ;
éthyle éther de POE (2) ;
éther de di (2 - hydroxypropyle)
pentane - 2,4 - diol ;
méthyle éther de POE (2) ;
propane -1- ol
1,4 - dioxane
dipélargonate de propylène glycol
éther stéarylique de polyoxypropylène 15 ;
alcool octylique ;
ester de POE d'alcool oléylique
alcool oléylique
alcool laurylique
adipate de dioctyle
adipate de dicapryle
adipate de diisopropyle
sébacate de diisopropyle
sébacate de dibutyle
sébacate de diéthyle
sébacate de diméthyle
sébacate de dioctyle
subérate de dibutyle
azélate de dioctyle
sébacate de dibenzyle
phtalate de dibutyle
azélate de dibutyle
myristate d'éthyle
azélate de diméthyle
myristate de butyle
succinate de dibutyle
phtalate de didécyle
oléate de décyle
caproate d'éthyle
salicylate d'éthyle
myristate d'isopropyle
palmitate d'isopropyle
laurate d'éthyle
pélargonate de 2 - éthyle - hexyle
isostéarate d'isobutyle
laurate de butyle
benzoate de benzyle
benzoate de butyle

laurate d'hexyle
caprate d'éthyle
caprylate d'éthyle
stéarate de butyle
salicylate de butyle
2 - pyrrolidone
1 - méthyle - 2 - pyrrolidone
5 - méthyle - 2 - pyrrolidone
1,5 - méthyle - 2 - pyrrolidone
1 - éthyle - 2 - pyrrolidone
alcool de tétrahydrofurfural
urée
diéthyle - m - toluamide ; et
1 - dodécylazacyloheptane - 2 - one.

**2.** Composition selon la revendication 1, dans laquelle l'huile non miscible dans l'eau comprend de l'huile minérale, des huiles végétales ou des huiles de silicone.

**3.** Composition selon la revendication 1 ou la revendication 2, dans laquelle le dérivé d'hydroxyalcanoate est un hydroxyalcanoate d'acyle, dans laquelle $R^1$ dans la structure (1) est représenté par le groupe acyle saturé ou insaturé :

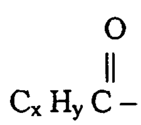

et dans laquelle :

$R^3$ représente - H ou - $CH_3$ ;
m va de 1 à 5 ; et
$R^3$ représente - H ou un métal alcalin, un métal alcalino-terreux, un ion antagoniste ammonium ou alcanol ammonium.

**4.** Composition selon la revendication 3, dans laquelle R1 est sélectionné à partir du :

n-propionyle
n-butanoyle
n-hexanoyle
n-décanoyle
n-dodécanoyle
n-tétradécanoyle
n-hexadécanoyle, et
n-ocatdécanoyle.

**5.** Composition selon la revendication 3 dans laquelle R1 dans la structure (1) représente le groupe acyle ramifié :

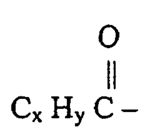

dans laquelle :

$C_xH_y$ - représente un groupe alkyle à chaîne ramifiée et m est 1.

**6.** Composition selon la revendication 5, dans laquelle R1 est sélectionné parmi :

l'iso - butanoyle
l'iso - hexanoyle
l'iso - octanoyle et
l'iso - octadécanoyle.

**7.** Composition selon les revendications 1 ou 2, dans laquelle le dérivé d'hydroxyalcanoate est un hydroxyalcanoate d'acyle dans lequel $R^1$ dans la structure (1) représente le groupe acyle saturé ou insaturé :

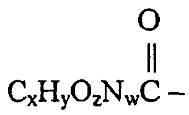

dans laquelle :

$R^2$ représente - H ou - $CH_3$ ;
m va de 1 à 5 ;
$R^3$ représente - H ou un métal alcalin, un métal alcalino-terreux, un ion antagoniste ammonium ou alcanol ammonium ;
z est 0, ou un nombre entier allant de 1 à 10 ;
w est 0, ou un nombre entier allant de 1 à 5, à condition que z et w ne représentent pas tous les deux 0 simultanément.

**8.** Composition selon la revendication 7, dans laquelle $R^1$ est sélectionné à partir de :

l'éthylglycoloyle
le leucoyle
le mandéloyle
le cocoamidopropanoyle
le pyroglutamoyle
le cholestéroyle et
le céramidoyle.

**9.** Composition selon la revendication 1 ou la revendication 2 dans laquelle le dérivé d'hydroxyalcanoate est un hydroxyalcanoate d'alkyle,
dans lequel :

$R^1$ représente H -
$R^2$ représente H - ou - $CH_3$
$R^3$ représente $C_xH_y$ - et
m est 1.

**10.** Composition selon la revendication 9, dans laquelle l'hydroxyalcanoate d'alkyle est sélectionné parmi :

le glycate de méthyle
le glycate de n - butyle
le glycate de n - hexyle
le glycate de n - octyle
le glycate de n - décyle
le glycate de n - dodécyle
le glycate de n - octadécyle
le lactate de méthyle
le lactate de n -butyle
le lactate de n - hexyle
le lactate de n - octyle

le lactate de n - décyle
le lactate de n - dodécyle
le lactate de n - tétradécyle
le lactate de n - hexadécyle
le lactate de n - octadécyle ; et
le lactate d'octyle décyle.

**11.** Composition selon l'une quelconque des revendications précédentes, dans laquelle la valeur de x est d'au moins 6.

**12.** Composition selon l'une quelconque des revendications précédentes dans laquelle la quantité de dérivé d'hydroxyalcanoate présent dans la composition en tant que quantité efficace, va de 0,001 à 50 %, de préférence de 0,1 à 30 %, et le plus préférentiellement de 0,5 à 20 % en poids de la composition.

**13.** Composition selon la revendication 12, dans laquelle la quantité de dérivé d'hydroxyalcanoate présent dans la composition va de 0,05 à 2 % en poids de la composition.

**14.** Composition selon l'une quelconque des revendications précédentes, dans laquelle le véhicule acceptable d'un point de vue cosmétique inclut l'eau dans une quantité qui est d'au moins 43 % de la composition et/ou inclut de l'éthanol aqueux dans une quantité qui est d'au moins 50 % en poids de la composition, la quantité d'éthanol, si celui-ci est présent, ne dépassant pas 50 % en poids de la composition.

**15.** Composition selon l'une quelconque des revendications précédentes, dans laquelle la composition comprend un rétinoide choisi parmi :

l'acétate de rétinyle,
le butyrate de rétinyle
le propionate de rétinyle
l'octanoate de rétinyle,
le laurate de rétinyle,
le palmitate de rétinyle,
l'oléate de rétinyle,
le linoléate de rétinyle, et
le linolénate de rétinyle ;

et/ou comprend du tocophérol et/ou de l'ester de tocophérol.

**16.** Composition selon l'une quelconque des revendications précédentes, dans laquelle la composition est un produit à laisser en place sélectionné parmi les crèmes, les lotions, les laits, les gels.

**17.** Compositions selon l'une quelconque des revendications précédentes, dans laquelle la composition comprend un tensioactif ionique autre que ledit dérivé d'hydroxyalcanoate.

**18.** Composition selon l'une quelconque des revendications précédentes, dans laquelle la composition est un produit avant rasage ou après rasage.

**19.** Composition selon l'une quelconque des revendications précédentes, dans laquelle la composition est appliquée sur le visage d'un être humain.

**20.** Utilisation d'un dérivé d'hydroxyalcanoate ayant la structure (1) comme défini dans l'une quelconque des revendications précédentes dans la fabrication d'une composition pour délivrer sur l'épiderme un 2 - hydroxyalcanoate ayant la structure (2) :

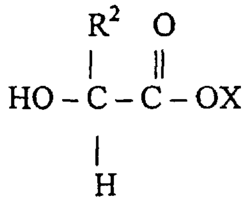

(2)

dans laquelle :

$R^2$ représente H - ou $CH_3$ ;
X représente H - ou un ion antagoniste,

comprenant les étapes consistant à :

(iv) appliquer de façon topique sur la peau la composition incluant le dérivé d'hydroxyalcanoate ayant la structure (1) ;
(v) laisser la composition en contact avec la peau pendant au moins 10 secondes afin de permettre au dérivé d'hydroxyalcanoate de pénétrer la couche cornée pour atteindre la couche inférieure de l'épiderme ; et
(vi) cliver le dérivé d'hydroxyalcanoate dans l'épiderme par contact avec les estérases de l'épiderme afin de fournir le 2 - hydroxyalcanoate ayant la structure (2).

Fig.1.

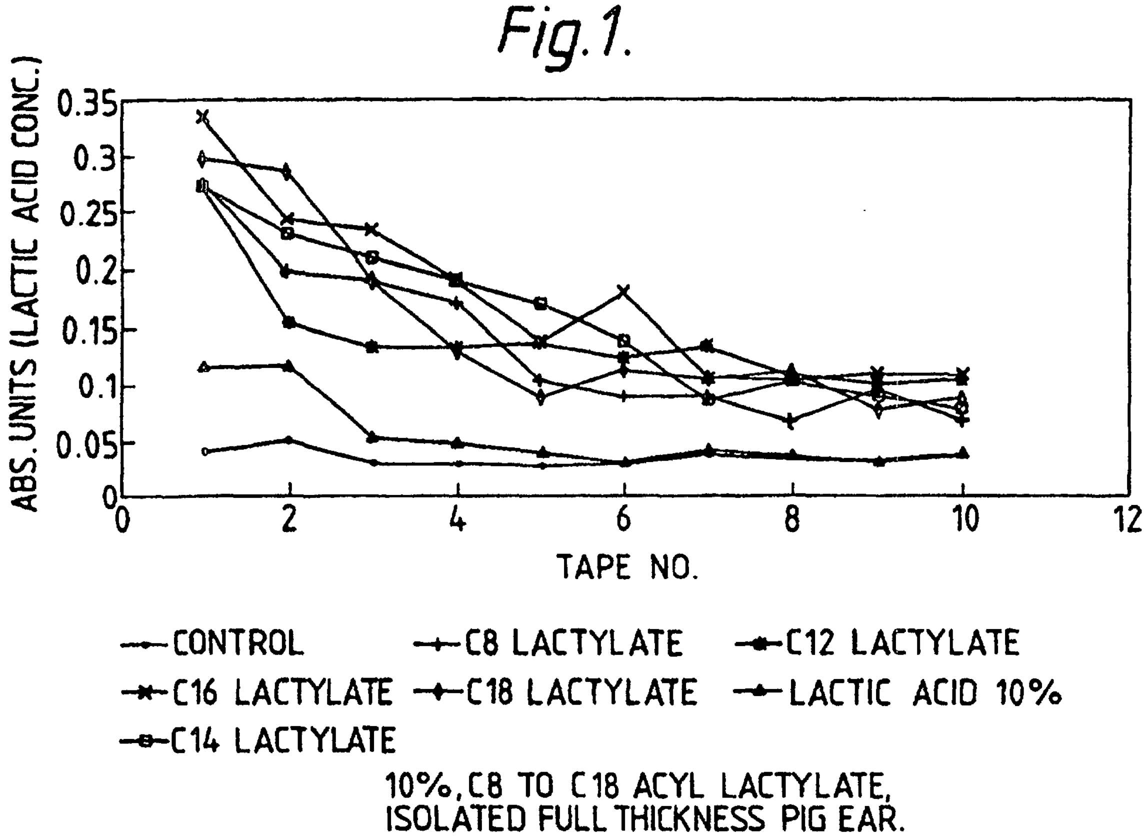
ABS. UNITS (LACTIC ACID CONC.)
0.35
0.3
0.25
0.2
0.15
0.1
0.05
0
0
2
4
6
8
10
12
TAPE NO.
CONTROL
C8 LACTYLATE
C12 LACTYLATE
C16 LACTYLATE
C18 LACTYLATE
LACTIC ACID 10%
C14 LACTYLATE
10%, C8 TO C18 ACYL LACTYLATE,
ISOLATED FULL THICKNESS PIG EAR.

Fig.2.

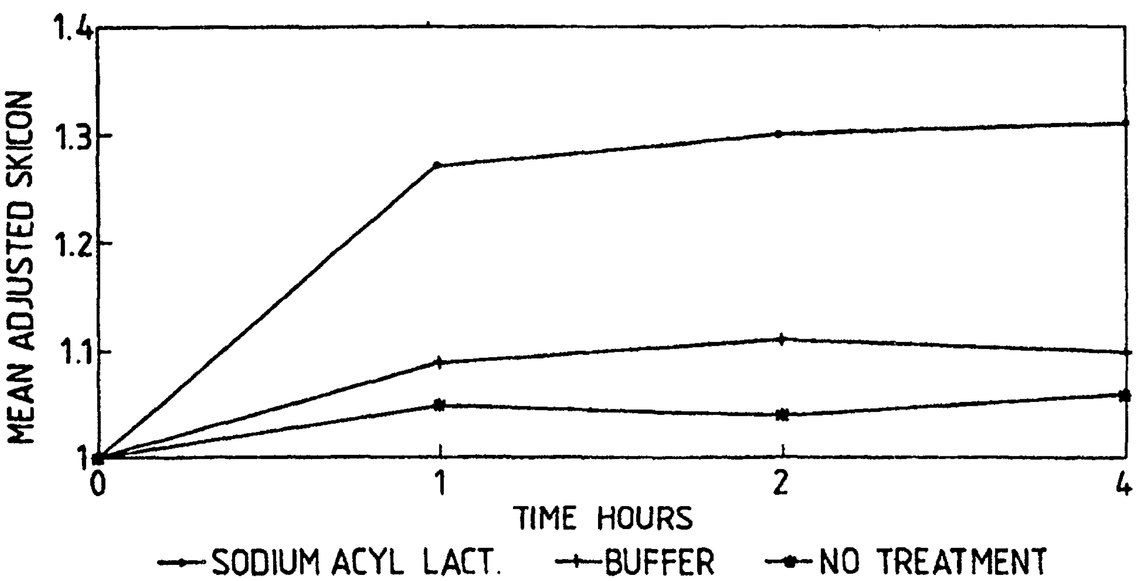
MEAN ADJUSTED SKICON
1.4
1.3
1.2
1.1
1
0
1
2
4
TIME HOURS
SODIUM ACYL LACT.
BUFFER
NO TREATMENT